(19) **Europäisches Patentamt**
**European Patent Office**
**Office européen des brevets**

(11) **EP 4 121 166 B1**

(12) # EUROPEAN PATENT SPECIFICATION

(45) Date of publication and mention
of the grant of the patent:
**31.01.2024  Bulletin 2024/05**

(21) Application number: **21710988.3**

(22) Date of filing: **15.03.2021**

(51) International Patent Classification (IPC):
*A61K 31/41* [(2006.01)]   *A61K 31/497* [(2006.01)]
*A61K 45/00* [(2006.01)]   *A61P 25/00* [(2006.01)]

(52) Cooperative Patent Classification (CPC):
**A61K 45/00; A61K 31/41; A61K 31/497**

(86) International application number:
**PCT/EP2021/056461**

(87) International publication number:
**WO 2021/185723 (23.09.2021 Gazette 2021/38)**

(54) **THERAPEUTIC TREATMENT OF CHROMATINOPATHIES**

THERAPEUTISCHE BEHANDLUNG VON CHROMATINOPATHIEN

TRAITEMENT THÉRAPEUTIQUE DE CHROMATINOPATHIES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **16.03.2020  IT 202000005527**

(43) Date of publication of application:
**25.01.2023  Bulletin 2023/04**

(73) Proprietor: **Università Degli Studi Di Trento
38122 Trento (IT)**

(72) Inventors:
• **ZIPPO, Alessio
38042 BASELGA DI PINE  (Trento) (IT)**

• **FASCIANI, Alessandra
21040 VENEGONO INFERIORE (Varese) (IT)**

(74) Representative: **Comoglio, Elena et al
Jacobacci & Partners S.p.A.
Corso Emilia 8
10152 Torino (IT)**

(56) References cited:
**EP-A1- 3 461 480     WO-A2-2016/112374**

Remarks:
The complete document including Reference
Table(s) and the Sequence Listing(s) can be
downloaded from the EPO website

**Description**

Field of the invention

The present invention relates to a compound for the therapeutic treatment of chromatinopathies, i.e. genetic disorders characterized by mutated genes encoding for chromatin regulators, including Kabuki Syndrome (KS), Kabuki Syndrome 2 (KS 2), Charge Syndrome (CS), Rubinstein-Taybi syndrome (RT) and Cornelia de Lange syndrome (CdL).

Description of the invention

Kabuki syndrome (KS) is a rare multisystemic genetic disorder characterized by craniofacial abnormalities, postnatal growth retardation, intellectual disability, various malformations of internal organs, as well as defects in the immune system. It is known to be caused by the haploinsufficiency of the KMT2D (50-75% of cases) or KDM6A (5-7%) genes, coding for the MLL4 and UTX proteins, respectively.

There are currently no therapeutic options to treat KS patients.

In an effort to understand the molecular basis of KS and to provide an effective treatment, the present inventors developed experimental models of the disease, both *in vitro* (mesenchymal stem cells, MSCs) and *in vivo* (Medaka fish), which allowed them to determine the consequences of KMT2D haploinsufficiency. Using these models, the inventors discovered the contribution of the MLL4 protein in establishing appropriate nuclear mechanical properties for cellular functionality. More in detail, the inventors demonstrated that the activity of the MLL4 protein encoded by the KMT2D gene consists in determining the chromatin compartmentalization in the nucleus. In addition, the inventors observed that the mutation of the KMT2D gene is sufficient to reduce the biological activity of the MLL4 protein, which causes an altered organization of the chromatin and affects the structural and mechanical properties of the nucleus. These alterations affect the ability of the affected cells to adequately respond to mechanical stimuli.

The inventors also discovered that targeting of the ATR protein (i.e., the "ataxia telangiectasia and Rad3-related protein") with specific drugs is sufficient to re-establish the adequate mechanical properties of the cells, with a consequent recovery of the phenotype involved. In particular, the inventors observed that the ATR inhibitor known as VX-970 (alternative names: VE-822; M6620, BERZOSERTIB; CAS Number: 1232416-25-9) is effective in blocking the activity of ATR at the level of the nuclear mechanics of the cells affected by mutations in the KMT2D gene that cause the Kabuki syndrome. This observation made it possible to provide an innovative therapeutic treatment for the Kabuki syndrome, for which there is currently no effective treatment available. Other specific inhibitors of the ATR protein known in the art and commercially available are readily usable as an alternative to VX-970 in the treatment of Kabuki syndrome. Examples of further specific inhibitors of the ATR protein include without limitation:

- BAY 1895344 (2-[(3R)-3-methylmorpholin-4-yl]-4-(1-methyl-1H-pyrazol-5-yl)-8-(1H-pyrazol-5-yl)-1,7-naphthyridine), CAS Number 1876467-74-1;
- AZD6738 (Ceralasertib), CAS Number 1352226-88-0;
- AZ20 (4-{4-[(3R)-3-Methylmorpholin-4-yl]-6-[1-(methylsulfonyl)cyclopropyl]pyrimidin-2-y 1}-1H-indole), CAS Number 1233339-22-4;
- EPT-46464 (2-Methyl-2-[4-(2-oxo-9-quinolin-3-yl-4H-[1,3]oxazino[5,4-c]quinolin-1-yl)phenyl]propanenitrile, 4-[4-(1-Isocyano-1-methyl-ethyl)-phenyl]-6-quinolin-3-yl-1,4-dihydro-2-oxa-4,9-diaza-phenanthren-3-one, CAS Number 1345675-02-6), CAS Number 1345675-02-6; and
- VE-821 (3-Amino-6-(4-(methylsulfonyl)phenyl)-N-phenylpyrazine-2-carboxamide, 3-Amino-6-[4-(methylsulfonyl)phenyl] -N-phenyl-2-pyrazinecarboxamide), CAS Number 1232410-49-9

It is also envisaged that inhibitors of Chkl, which is an ATR effector protein, shall also be effective in the treatment of Kabuki syndrome. Non-limiting examples of inhibitors of Chkl are:

- GDC-0575 (ARRY-575, RG7741), CAS Number 1196541-47-5
- - AZD7762, CAS Number 860352-01-8
- - MK-8776 (SCH 900776) CAS Number 891494-63-6
- - SAR-020106, CAS Number 1184843-57-9
- - CCT245737, CAS Number 1489389-18-5
- - PF-477736 (PF-736,PF-00477736) CAS Number 952021-60-2

Furthermore, since the Kabuki syndrome is part of a group of genetic disorders sharing similar clinical pictures and causative mutations in genes coding for chromatin regulators, it is envisaged that the aforementioned specific inhibitors of the ATR protein will be effective against all of such genetic disorders, which are collectively designated as chromatinopathies and include Kabuki Syndrome (KS), Kabuki Syndrome 2 (KS 2), Charge Syndrome (CS), Rubinstein-Taybi syndrome (RT) and Cornelia de Lange syndrome (CdL).

To the inventors' knowledge, it is the first time that inhibitors of the ATR protein are demonstrated to be effective in the treatment of chromatinopathies. Such drugs are in fact currently being tested for the treatment of various tumor diseases. For example, VX-970 is currently under evaluation for its therapeutic activity against different types of tumors, in 14 different experimentations including both Phase I and II clinical studies.

Accordingly, an aspect of the present invention is an inhibitor of the Ataxia Telangiectasia and Rad3-related (ATR) protein or of the ChkI protein for use in the therapeutic treatment of a chromatinopathy.

In a preferred embodiment of the invention, the chromatinopathy is selected from the group consisting of Kabuki Syndrome (KS), Kabuki Syndrome 2 (KS 2), Charge Syndrome (CS), Rubinstein-Taybi syndrome (RT) and Cornelia de Lange syndrome (CdL).

In another preferred embodiment of the invention, the inhibitor of the ATR protein is selected from the group consisting of VX-970, BAY 1895344, AZD6738, AZ20, EPT-46464 and VE-821.

In a further preferred embodiment of the invention, the inhibitor of the ChkI protein is selected from the group consisting of GDC-0575, AZD7762, MK-8776, SAR-020106, CCT245737 and PF-477736.

A particularly preferred embodiment of the invention is the ATR protein inhibitor designated as VX-970 for use in the therapeutic treatment of the Kabuki syndrome.

Other features and advantages of the invention will become apparent from the following examples, which are provided by way of illustration only and is not intended to limit the scope of the invention as defined by the appended claims.

The examples illustrate in detail the experiments carried out by the present inventors, which demonstrated the following:

(i) In Kabuki syndrome, haploinsufficiency of KMT2D causes loss of function of the MLL4 protein, which consequently causes alterations in the ability to compartmentalize chromatin.

(ii) The molecular mechanisms that cause the aberrant compartmentalization of chromatin result in an altered balance between the MLL4 and Polycomb chromatid complexes. This altered chromatin organization causes an increase in nuclear mechanical forces in the cells, causing hyperactivation of ATR and its downstream responses.

(iii) The targeting of ATR with the specific inhibitor VX-970 is sufficient to restore the mechanical properties of the treated mesenchymal stem cells (MSCs), as measured by the cellular distribution of the YAP1 mechanical-effector protein whose nuclear accumulation depends on the mechanical forces.

(iv) By analyzing gene expression in response to the treatment with VX-970, the ATR-sensitive genes that belong to the functions of chromatin architecture were identified. Considering that mesenchymal stem cells (MSCs) respond to mechanical signals to direct their cellular fate during differentiation, the inventors showed that the loss of function of MLL4 compromises the cell differentiation towards the osteocytic and chondrocyte lines, which give rise to bones and cartilages. The treatment of MSCs with VX-970 is sufficient to abolish the differentiation defects, restoring the correct chondrogenesis *in vitro.*

(v) To verify the therapeutic potential of the aforementioned approach, the inventors developed an *in vivo* model of KS, reducing the Kmt2d expression levels in Medaka (a vertebrate fish experimental model used for studying genetic diseases). The Medaka model phenocopies the craniofacial dysmorphism that affects KS patients with perturbations in chondrogenesis and ossification. In this context, the inventors showed that the treatment of Medaka affected by Kmt2d haploinsufficiency with VX-970 was sufficient to completely recover the chondrogenic and osteogenic defects, without side effects.

Taken together, the results obtained by the present inventors demonstrate that the inhibition of ATR represents an extremely promising therapeutic option for Kabuki syndrome and other rare genetic diseases caused by the haploinsufficiency of genes that code for chromatin factors functionally related to MLL4, including Kabuki Syndrome 2 (KS 2), Charge Syndrome (CS), Rubin-stein-Taybi syndrome (RT) and Cornelia de Lange syndrome (CdL).

**EXAMPLES**

**Methods**

Cell culture conditions

Cell lines used in this study include NIH 3T3 (ATCC), HEK293T (ATCC), human primary fibroblasts derived from either healthy of Kabuki patients (Genomic and Genetic Disorders Biobank) and hTERT-immortalized human adipose-derived MSCs. Primary fibroblasts, NIH3T3 and HEK293T were maintained at 37°C and 5% $CO_2$ in DMEM medium supplemented with 10% Fetal Bovine Serum (Euroclone #ECS0180L), while MSCs were cultured in 1:1 DMEM/F-12 medium (Gibco #11320-074) supplemented with 10% Fetal Bovine Serum (Euroclone #ECS0180L).

For adipocyte differentiation, cells were seeded with a density of $1 \times 10^4$ cells/cm$^2$ in MSCs medium. The day after, medium was changed with adipogenesis medium (Gibco #A10410-01) supplemented with Stem-Pro Adipogenesis supplement (Gibco #10065-01). For complete differentiation, the cells were maintained in culture for three weeks changing media regularly.

For osteoblasts differentiation, cells were seeded with a density of $5 \times 10^3$ cells/cm$^2$ in MSCs medium. The day after, the medium was changed with osteogenesis medium (Gibco #A10069-01) supplemented with Stem-Pro Osteogenesis supplement (Gibco #10066-01). For complete differentiation, the cells were maintained in culture for three weeks changing media regularly.

For chondrocyte micromass culture, a cell solution of $1.6 \times 10^7$ viable cells/mL was produced. Micromass cultures were generated by seeding 5-$\mu$L droplets of cell solution. After cultivating micromass cultures for 2 hours under high humidity conditions, MSCs medium was added. The day after, the medium was changed with chondrogenesis medium (Gibco #A10069-01) supplemented with Stem-Pro Chondrogenesis supplement (Gibco #10064-01). For complete differentiation, cells were maintained in culture for three weeks changing media regularly.

CRISPR/CAS9 genome editing

sgRNAs were designed using the online tool e-Crisp (Boutros lab, E-CRISP-Version 5.4, http://www.e-crisp.org/E-CRISP/). Once designed, they were cloned in the pLX sgRNA vector (from Addgene, #50662). Briefly, new target sequences were cloned into pLX sgRNA between the XhoI and NheI sites using overlap-extension PCR followed by restriction/ligation into pLX sgRNA vector. MSCs were genome edited by expression of the doxycycline-inducible Cas9 (pCW Cas9, Addgene #50661) combined with sgRNA construct, followed by puromycin and blasticidin selection. Clonal selection was performed to identify targeted cells. Genomic DNA was collected from different clones and subjected to surveyor assay (using the T7 endonucleases, NEB #M0302). Positive clones were selected and sequenced to determine the insertion of the truncating mutation. The oligonucleotides used in this work for the generation of sgRNAs containing plasmids are listed in Table 1.

Table 1

| Oligonucleotides used for genome editing | |
|---|---|
| NAME | SEQUENCE |
| sgRNA-F1 | aaactcgagtgtacaaaaaagcaggctttaaag |
| sgRNA-F2 | gtggctgcagctgcagagagcgttttagagctagaaatagcaa |
| sgRNA-R1 | gctctctgcagcrgcagccacggtgtttcgtcctttcc |
| sgRNA-F2 | gtggctgcagctgcagagagcgttttagagctagaaatagcaa |
| | |
| **Oligonucleotides used for cloning** | |
| NAME | SEQUENCE |
| MLL4 PrLD_fwd_BglII | GCCAGATCTGGTGATGCTGAGAAGCTCAAGCT |
| MLL4 PrLD_rev_SalI_STOP | GCCGTCGACTTTACTGTGGTCCAGGGAAGCC |
| MLL4-PrLD ΔQ_revi_ | TAAAGAGCCCATGGGCTGAGCGCTCAGTT |
| MLL4-PrLD ΔQ_fwdi | GCTCAGCCCATGGGCTCTTTTTTTTAAACCAGAGTCGAACTTTACTGTCTC |
| | |
| **Oligonucleotides used for gene expression analysis** | |
| NAME | SEQUENCE |
| GAPDH fwd | AGGTGAAGGTCGGAGTCAAC |
| GAPDH rev | CCATGTAGTTGAGGTCAATGAAG |
| RING1B fwd | GCCAGGATCAACAAGCACA |
| RING1B rev | TTGTTTCTTGCCTCGCTGC |

(continued)

| Oligonucleotides used for gene expression analysis | |
|---|---|
| NAME | SEQUENCE |
| KDM6A fwd | CCTCTTTGGGTTCGTGAGAT |
| KDM6A rev | GACTCCACTTTTCCTTCAGCTT |
| BMI fwd | TCATTGTCTTTTCCGCCCG |
| BMI rev | TCAGGTGGGGATTTAGCTCA |
| KMT2D fwd | tgaaagggcactgagggata |
| KMT2D rev | tgagggggtgtaggcaag |
| LMNA fwd | ACCCATCTCCTCTGGCTCTT |
| LMNA rev | GTGACCAGATTGTCCCCGAA |
| TOP2B fwd | AGGAAAGCATCTGGCTCTGA |
| TOP2B rev | TCTGAGGGGAAGATGTCCAC |
| SMC4 fwd | TCGGTTCTAAGCCCAGAGGA |
| SMC4 rev | TATACTCTGCGATGGCACCG |
| RAD21 fwd | TCCCCCAGAAGAACCTCCAA |
| RAD21 rev | ACGCTGAAGACCATGAAGCA |
| TOP2A fwd | TGTCAAACCAGTTCCTGCAA |
| TOP2A rev | CTGTGACGAAACCATGTTGG |
| COL II fwd | CAGGATGTCCAGGAGGCT |
| COL II rev | GCTTCCACACATCCTTATCATT |
| AGC fwd | GGAGTCCAACTCTTCAAGGTG |
| AGC rev | ATGGTCTGAAGTTTCTACAGTGACA |
| COL10A1 fwd | CCCAACACCAAGACACAGTT |
| COL10A1 rev | GTGGACCAGGAGTACCTTGC |
| MMP13 fwd | GCAGCTGTTCACTTTGAGGA |
| MMP13 rev | CATCATATCTCCAGACCTGGTT |
| | |
| Morpholino sequence | |
| NAME | SEQUENCE |
| MO-Kmt2d | CCCTGCTGCTGCTTTGATCTTTTTG |

Immunohistochemical analysis

To detect adipogenesis, cells were washed with PBS and fixed in 4% formaldehyde for 1 hour at RT, washed with PBS and then stained for 1 hr with fresh and filtered Oil-Red O solution (Sigma-Aldrich # O0625) composed of 3 parts of a 0.5% stock solution in isopropanol and 2 parts of distilled water. Then cells were washed three times with distilled water.

To detect ostogenesis, cells were washed with PBS, fixed with ice-cold 70% ethanol and incubated with filtered 2% (p/v in distilled water) Alizarin red solution (Sigma # A5533) for 15 min. Then cells were washed three times with distilled water.

To detect chondrogenesis, cells were washed with PBS and fixed in 4% formaldehyde for 1 hr at RT and then washed with PBS. Cells were incubated with Alcian blue solution (1 g/L in 0.1 M HCl, Sigma-Aldrich # B8438) for 6 hrs at room temperature and then extensively washed with PBS. To measure Alcian blue deposition, dry wells were incubated with 1 mL of 6 N Guanidine HCl for 1 hour and then the absorbance was measured with a spectrophotometer measured between 600 and 650 nm.

DNA constructs

The mCherry-Cry2 sequence was PCR amplified from the pHR-mCherry-MED1_IDR-CRY2, and cloned between the XhoI and NotI sites in the pCAG vector with or without the insertion of a SV40 NLS al the 3' of CRY2. The MLL4 PrLD region (from amino acid 3560 to 4270) was PCR amplified and cloned between the XhoI and NheI sites in the expression vector pCAG mCh-CRY2-NLS. The MLL4 PrLD ΔQ region was obtained by overlap-extension PCR and cloned between the XhoI and NheI sites in the pCAG mCh-CRY2-NLS vector. The mouse BMI coding sequence was PCR amplified and cloned between the EcoRV and SpeI sites in the expression vector pCAG mCh-CRY2. The oligonucleotides used for cloning are listed in Table 1.

Stable cell lines

MSCs expressing mCh-MED1-CRY2 were obtained by transducing WT and mutant MSCs with the lentiviral vector pHR mCh-MED1-Cry2. MSCs expressing the H3.3K27M were obtained by transducing mutant MSCs with the lentiviral vector pCDH-EF1-MCS-IRES-PURO-H3.3K27M. MSCs overexpressing YAP were obtained by transducing mutant MSCs with the lentiviral vector FUW tetO YAP (Addgene #84009).

Immunofluorescence

For immunofluorescence assays, cells were seeded on coverslips coated with 0,1% gelatin (Sigma Aldrich # G1393). When needed, cells where fixed with 4% paraformaldehyde for 10 minutes at 4°C. Coverslips were processed as described: permeabilization and blocking with PBS/1% BSA/5% goat serum/0.5% Triton X-100 (blocking solution) for 1 hour at room temperature, followed by incubation with primary antibody (diluted in the blocking solution) for 2 hours at RT (or overnight at 4°C, depending on the used primary antibody), 3-5 washes in PBS and incubation with secondary antibodies (diluted in the blocking solution), DRAQ5 for nuclear staining and phalloidin-TRITC for 1h at room temperature. Images were acquired using a Leica TCS SP5 confocal microscope with HCX PL APO 63x/1.40 objective. Confocal z stacks were acquired with sections of 0.5 μm. In cases where image analysis was performed, image acquisition settings were kept constant. The antibodies used are listed in Table 2.

Table 2

| ANTIBODY | | | |
|---|---|---|---|
| Target | Company | Code | Application |
| MLL4 | Sigma | HPA035977 | IF/WB |
| UTX | Cell Signaling | 33510 | IF/WB |
| PA1 | Bethyl | A301-978A | IF/WB |
| WRD5 | Bethyl | A302-430A | IF/WB |
| Histone H3 | Cell Signaling | 9715 | IF/WB |
| BRD4 | abcam | ab128874 | IF/WB |
| MED1 | abcam | ab64965 | IF/WB |
| BMI1 | Millipore | 05-637 | IF/WB |
| RING1B | Cell Signaling | 5694 | IF/WB |
| Lamin A | Santa Cruz | sc-71481 | IF/WB |
| phospho-Lamin A/C (Ser22) | Cell Signaling | 13448 | IF/WB |
| EED | Millipore | 05-1320 | IF/WB |
| EZH2 | Cell Signaling | 3147 | IF/WB |
| SUZ12 | Cell Signaling | 3737 | IF/WB |
| H3K4me1 | Abcam | ab8895 | IF |

(continued)

| ANTIBODY | | | |
|---|---|---|---|
| Target | Company | Code | Application |
| H3K27ac | Abcam | ab4729 | IF |
| H3K27me3 | Millipore | 07-449 | IF/WB |
| H4K16ac | Millipore | 06-762 | IF |
| YAP/TAZ | Cell Signaling | 8418 | IF/WB |
| YAP | Santa Cruz | sc-271134 | WB |
| YAP1 (phospho Ser127) | GeneTex | GTX130424 | WB |
| pH2AX | Cell Signaling | 9718 | IF |
| ATR | Cell Signaling | 2790 | IF |

Confocal imaging data analyses

Confocal imaging data analyses were performed using Image J software. For 2D/3D analysis RDAQ5 DNA dye was used to identify the nucleus and define the ROI. Then the fluorescence intensity and physical parameters were determined. For the measure of volume and flatness the inventors performed a 3D analyses using the "3D plugin 596 suite", an Image J plugin.

Quantification of Nuclear to cytosolic localization of YAP/TAZ

To quantify the nuclear to cytosolic localization of YAP-TAZ, the inventors adapted previously published MATLAB routines (Zambrano et al., 2016). Adapted routines are available upon request. In short, images of the Hoechst and YAP TAZ channels were saved as 16-bit tiffs files. To segment the nuclei, the inventors used the signal from the Hoechst channel. The nuclear masking was performed using as a threshold the mean intensity of the image plus twice the standard deviation. After thresholding, segmentation was carried out after a watershed transformation, so most of the few overlapping nuclei could be separated. The segmented nuclei were filtered by size a posteriori to exclude artifacts or improperly segmented clusters of nuclei.

To estimate the average cytosolic intensity per cell, a ring of 30 pixels width (approximately 7 microns) around each segmented nuclei was found. Pixels of the ring with too low intensity of the YAP-TAZ signal (below twice the value of the background signal) are discarded. The average cytosolic signal for each cell is the average intensity of the remaining pixels. The inventors then calculated for each cell the Nuclear to Cytosolic Intensity (NCI) as the ratio of the background corrected nuclear and cytosolic average YAP-TAZ intensity.

3D Imaging cluster data analyses

In order to automatically detect and quantify PcG and TrxG complex proteins in fluorescence cell image z-stacks, the inventors developed an algorithm that implements a method derived from (Gregoretti et al., 2016) with variants and adaptations. The algorithm performs the 2D segmentation of cell nuclei and the detection of Protein Bodies (PBs) for each slice of the stack, followed by the 3D reconstruction and identification of nuclei and PBs. It then measures the volume of nuclei and the number and volume of the PBs and the relative positioning of PBs in the nucleus. The algorithm has been implemented in MATLAB following this scheme:

$$t_{temp} = -255;$$

for each slice n of the stack

$$I_{dapi+} = (I_{dapi} + \mathit{medfilt2}(I_{PBfluo,n}\,[4,4]))/2$$

%Performs 2D nuclei segmentation

$$[\text{nuclei}_n, \text{avg}_{\text{PBfluo},n}] = \boldsymbol{nuclei\_seg}(\text{I}_{\text{dapi+},n}, \text{I}_{\text{PBfluo},n})$$

$$\text{nuclei\_vol}(:,:,n) = \text{nuclei}_n(:,:)$$

$$\text{I}_{\text{avg},n} = \boldsymbol{imfilter}(\text{I}_{\text{PBfluo},n}, \boldsymbol{fspecial}(\text{'average'}, [3,3]))$$

%Identifies PB regions with highest intensity

$$\text{I}_{\text{filt},n} = \text{I}_{\text{PBfluo},n} - \text{I}_{\text{avg},n}$$

%Evaluates threshold for PBs detection

$$\text{t}_{\text{temp}} = \boldsymbol{isodata\_thresh}(\text{I}_{\text{filt},n}, \text{avg}_{\text{PBfluo},n})$$

$$\text{t}_{\text{max}} = \max(\text{t}_{\text{max}}, \text{t}_{\text{temp}})$$

endfor
for each slice n of the stack
%Performs 2D PBs detection

$$\text{PBs}_n = \text{I}_{\text{filt},n} > \text{t}_{\text{max}}$$

$$\text{PBs\_vol}(:,:,n) = \text{PBs}_n(:,:)$$

endfor

$$\text{nuclei\_CC} = \boldsymbol{bwconncomp}(\text{nuclei\_vol})$$

$$\text{nuclei\_L} = \boldsymbol{labelmatrix}(\text{nuclei\_CC})$$

compute volume for each nucleus object in nuclei_CC
exclude nuclei whose volume is less than 10% of mean volumes

$$\{\text{NCL}\}\text{M} \leftarrow \text{identified 3D nuclei}$$

for each nucleus m in {NCL}$_M$

$$\text{NCL}_m.\text{PBs} = \text{PBs\_vol} .* \{\text{NCL}\}\text{M} \quad \text{\%3D positions of detected PBs within the nucleus}$$

NCLm

$$\text{NCL}_m.\text{PBs} = \boldsymbol{bwareaopen}(\text{NCL}_m.\text{PBs}, 17, 6);$$

$$\text{PBs\_CC} = \boldsymbol{bwconncomp}(\text{NCL}_m.\text{PBs})$$

compute number of PBs in PBs_CC
compute volume for each PB in PBs_CC
compute distances of any PB from the nuclear periphery
compute distances of any PB from the nuclear centroid

endfor

$I_{dapi}$ is the image showing the fluorescence of nucleus while $I_{PBfluo}$ is the image showing the fluorescence of PBs. *PBfluo* stands for BMI11 or RING1B in case of PcG and BRD4 or MLL4 in case of TrxG. In order to better enhance nucleus areas the inventors added to $I_{dapi}$ the image obtained by performing a median filtering of $I_{PBfluo}$, producing the image $I_{dapi+}$.

The function *nuclei_seg* performs a partition of cell image $I_{dapi+}$ in nuclei regions and background implementing a region based segmentation algorithm (Goldstein et al., 2010), and computes $avg_{PBfluo}$, the mean intensity value of the nuclei regions in the image $I_{PBfluo}$.

In order to better enhance PB areas the inventors subtract from the original $I_{PBfluo}$ image its smoothed version obtained by applying an averaging filter of size 3, producing the image $I_{filt}$.

The function *isodata_thresh* implements the ISODATA classification algorithm and uses relevant values computed by *nuclei_seg* function in order to extract PBs from the nuclei regions. It sets the initial threshold value of ISODATA method as $avg_{PBfluo}$.

For each slice of the stack, the algorithm separates PBs from nuclei regions by means of a thresholding operation using the maximum of the threshold values estimated by the function *isodata_thresh* applied to all the images $I_{filt,n}$.

*PBs_vol* and *nuclei_vol* are 3D arrays that contain the positions of the detected PBs and nuclei from all slices.

The 3D reconstructions of nuclei are obtained through the connected components algorithm (*bwconncomp* MATLAB function, using a connectivity of 26). 3D nuclei are then labeled by applying the *labelmatrix* MATLAB function so they can easily separate each from the others.

The algorithm computes the volume of each 3D reconstruction, discarding objects whose volume is less than 10% of mean volumes which are just noise.

The algorithm uses the *bwareaopen* function in order to discard too small (less than 17 pixels) detected PB objects which are probably just noise.

3D reconstructions of PBs are obtained through the connected components algorithm (*bwconncomp* MATLAB function, using a connectivity of 6).

The algorithm computes the number of PBs, the volume of any PB and the distances of the centroid of each PB from the nuclear periphery and the nuclear centroid.

Super-resolution microscopy

Super-resolution localization imaging of fixed and immunostained cells was obtained by direct stochastic optical reconstruction microscopy (dSTORM), using a GSD microscope (Leica SR GSD, Leica M icrosystems, Mannheim, Germany) equipped with two solid state lasers of 532 nm and 642 nm, an oil immersion objective lens (HCX PL APO 150x 1.45NA), and an EMCCD camera (Andor iXon Ultra-897). All dSTORM experiments were performed with the Smart-kit buffer (Abbelight, France). To induce the majority of the fluorophores into the dark state, the inventors excited the samples using the laser in a straight configuration. Once the density of fluorescent dye was sufficient, the inventors activated the real-time localization using the laser in an oblique configuration (Hilo). For all recorded images, the integration time and the EMCCD gain were set to 8 ms and 300, respectively. For each cell where acquired 35000 frames. The identification and localization of single events from raw images was run on the Leica software.

STORM images data analysis

The cluster analysis was performed with a custom-written Matlab script following the routine described in (Ricci et al., 2015). Briefly, for each cell the localizations list was used to reconstruct a STORM image with pixel size of 20 nm. This image was used to exclude areas of very low localization density (density threshold = $0.0025$ nm$^{-2}$) and to identify the local maxima in the areas of higher density. Only localizations within high density regions are analysed. The number and position of the maxima are used to initialize the centroids of the clusters. The subdivision of the localizations in clusters is performed by a machine learning k-mean algorithm which optimizes the grouping of localizations based on their proximity to the centroid of the cluster. The algorithm runs on the raw localizations coordinates. The area attributed to the cluster is the convex hull area associated to that set of localizations.

Live Imaging and Optogenetics

Time-lapse video microscopy and single-cell tracking of MSCs and NIH3T3 were carried out continuously for the indicated time at 37 °C and 5% $CO_2$, using the Eclipse Ti2 fully automated system (Nikon). Images of fluorescent cells were acquired every 10 seconds for short time-lapse experiments or every 20 minutes for long time-lapse experiments with 100 × or 60x Plan Apo λ objective (Nikon) using a LED illumination system combined with a CMOS camera (Andor) for the detection. Single-cell tracking was performed using the NIS software and movies were assembled using Image J software.

Live imaging data analysis

For the analysis of the optogenetics experiments, the NIS software was used.
For the MED1 clusters, a single nucleus analysis was performed. For each nucleus background correction and Gauss-Laplace sharpen filter was applied. A threshold was set such that clusters are identified after the stimulation. A single-cluster tracking was performed and the area of each cluster was determined.
For the MLL4_PrLD and for the BMI clusters, a single nucleus analysis was performed. For each nucleus background correction and Gauss-Laplace sharpen filter was applied. The "bright spot detection" function was used to identify the single clusters. The threshold parameter was determined in order to identify as individual objects clusters in close proximity to one another.

Protein Extraction and Western Blot Analysis

For histone modifications, total protein extracts were obtained as follows. Cells were washed twice with cold PBS, harvested by scrapping in 1 ml cold PBS and centrifuged for 5 minutes at 1500 rpm. Pellet was resuspended in acid buffer (10mM Hepes pH 8, 10mM KCl, 0.1mM $MgCl_2$, 0.1mM EDTA pH 8, 2mM PMSF, 0.1mM DTT) in order to have $10^7$ cells/ml. Cells were left at 4°C 10 minutes and then were centrifuged for 10 minutes at 5000 rpm at 4°C. The supernatant (the citosolic extrac) was discarded and the pellet was resuspended in 0.2N HCl in order to have $4\times10^7$ cells/ml and left O/N at 4°C on rotating wheel. The day after, proteins were recovered by centrifugation for 10 minutes at 4000rpm at 4°C. Supernatant was recovered and protein concentration was measured with Bradford assay (Biorad #5000006) according to manufacturer's instructions.
Nuclear protein extracts were prepared in hypotonic buffer (Tris-HCl 50 mM; NaCl 137.5 mM; 1% NP-40; EDTA 5 mM; 10% Glycerol; 0.5% Triton; 0.5% SDS). Harvested cell pellets were lysed by the addition of 6× v/v ice-cold hypotonic buffer w/o SDS for 15 min at 4 °C. The supernatant containing the cytoplasmic fraction was collected and stored, by centrifugation for 5 min at 100 xg, at 4 °C. After two washes in hypotonic buffer, nuclear pellets were resuspended in 6× v/v ice-cold complete cell extraction buffer and sonicated. Lysates were cleared by centrifugation for 10 min at 21000 xg at 4°C and supernatant was collected. Western Blots were performed using the antibodies listed in Table 2.

Recombinant protein purification

pET-mCherry-MLL4-PrLD/ PrLD ΔQ was subcloned from pET mCherry-MED1-IDR. Briefly, the MLL4 PrLD region (from amino acid 3560 to 4270) was PCR amplified and cloned between the BglII and SalI sites in the pET mCherry-MED1 IDR. The MLL4 PrLD ΔQ region was obtained by overlap-extension PCR and cloned between the BglII and SalI sites in the pET mCherry-MED1 IDR. The Protein purification was done using a standard protocol as follow: bacterial pellet was resuspended in 25mL of Ni-NTA Lysis Buffer (LB) (50 mM TrisHCl pH 7.5, 500mM NaCl), and sonicated. The lysate was cleared by centrifugation at 12,000g for 20 minutes at 4°C and added to Ni-NT Agarose (Qiagen, ID: 30210) pre-equilibrated with Ni-NTA LB. Tubes containing the agarose lysate slurry were rotated at 4°C for 1 hour. The agarose beads were collected by centrifugation for 5 min at 200 g and were transferred to the gravity columns. The protein-bound beads were further washed with the Ni-NTA LB containing 10mM Imidazole. Protein was then eluted with Ni-NTA LB containing 50/100/250 mM imidazole. The proteins were further purified over the gel filtration chromatography (Superdex 200 Increase 10/300 GL, GE Healthcare #28990944) and equilibrated with Buffer D (50mM Tris-HCl pH 7.5, 766 125mM NaCl, 1mM DTT, 10% glycerol). Peak fractions were pooled, aliquoted and concentrated using Pierce™ Protein Concentrator PES, 10K MWCO (Thermo Scientific™ #88527). The eluted fractions containing protein were finally analyzed by Comassie stained gel.
*In vitro* phase separation assay was performed as previously described in (Sabari et al., 2018). Briefly, recombinant protein was added to Buffer D containing 10% Polyethylene glycol (PEG) 8000 (Sigma # 1546605) at varying concentrations with indicated final salt and 1.6- hexanediol (Santa Cruz #sc-237791). The protein solution was immediately spotted into a glass slide and then covered with a coverslip. The solution was allowed to mix for 5 minutes at room temperature followed by imaging acquisition. Images of formed droplets were acquired using a Zeiss Axio Observer inverted microscope with an AxioCam 503 mono D camera and a Plan-Apochromatic 100x/1.4 oil-immersion objective equipped with prism for DIC (Zeiss).

Images were analyzed with FIJI image processing package (http://fiii.sc/). The inventors determined the intensity signal inside and outside the droplets by setting a threshold on the minimum intensity observed at the lower tested concentration where droplets formed, then this threshold was applied to every condition. The saturation concentration was quantified as previously described in (Wang et al., 2018). The inventors measured the fluorescence intensity inside the droplets (Idroplet) and the fluorescence intensity outside the droplets (Imedia) by summing respectively the intensity of each pixel inside and outside droplets. The amount of condensed protein for a given candidate under a certain concentration is defined by the ratio of Idroplet to Imedia. If no droplets are present for a certain condition the ratio is set to zero. Condensed protein appears only above the saturation concentration.

## Quantification of Histone PTMs by LC-MS/MS

Histone samples (20ug) were suspended in 50 mM $NH_4HCO_3$ and subjected to chemical derivatization and digestion as previously described (Sidoli et al., 2016). Briefly, propionic anhydride solution was freshly prepared by mixing propionic anhydride with acetonitrile in the ratio 1:3 (v/v), creating the propionylation mix. Next, propionylation mix was added to the histone sample in the ratio of 1:4 (v/v), immediately followed by $NH_4OH$ with a ratio of 1:5 (v/v) to adjust the pH to ~8.0. Samples were incubated for 15 min at 37°. Propionylation was repeated a second time after drying samples in a SpeedVac centrifuge. Samples were dried, dissolved in 50 mM $NH_4HCO_3$ and digested overnight with trypsin at an enzyme:sample ratio of 1:20. The digested peptides were then treated with an additional round of propionylation for the derivatization of peptide N-termini. After drying in a SpeedVac, the samples were desalted by C18 stage-tip, lyophilized, and resuspended in 20 $\mu$l of 0.1% formic acid for LC-MS/MS analysis.

Samples were analyzed using an EASY nLC 1200 ultra-high pressure liquid chromatography system (Thermo Scientific) coupled to an Orbitrap Fusion mass spectrometer (Thermo Scientific). Briefly, 1 $\mu$g of sample was loaded on a 25 cm long Acclaim PepMap RSLC C18 column (Thermo Fisher Scientific, 2$\mu$m particle size, 100Å pore size, id 75 $\mu$m) heated at 40°C. Mobile phase A was 0.1% formic acid, mobile phase B was 80% acetonitrile/0.1% formic acid (v/v). The gradient was as follows: from 2 to 34%B over 45 min, from 34 to 90%B in 5 min, and 90%B for 10 min at a flow rate of 300 nl/min. MS acquisition was performed using a data-dependent acquisition (DDA) mode.

To quantify of histone PTMs, raw files obtained from the LC-MS runs were processed using EpiProfile, a software tool that performs extracted ion chromatography (XIC) of histone with a peak extraction mass tolerance set to 10 ppm. Once the peak area was extracted, the relative abundance of a given PTM was calculated by dividing its intensity by the sum of all modified and unmodified peptides sharing the same amino acid sequence.

## Luciferase Assay

YAP/TAZ activity was determined by luciferase reporter assays. WT and $MLL4^{Q4092X}$ MSCs were nucleofected with either the YAP/TAZ responsive luciferase reporter plasmid (8xGTIIC-luciferase, from Addgene #34615) or the empty vector (pGL4.27[luc2P/minP/Hygro]) and the pGL4-CMV-Renilla luciferase vector as a normalization control in a 30:1 ratio. $1 \times 10^6$ cells were nucleofected using an Amaxa Nucleofector (program U-23, Lonza) and homemade buffer (KCl 5 mM, $MgCl_2$ 15mM, Glucose 1M, $K_2HPO_4$ 120mM). After 24 hours of incubation, nucleofected cells were re-seeded as sparse (5000 cells/cm$^2$). After 24 hours, Firefly and Renilla luciferase activity was measured using the Dual-Luciferase® Reporter Assay System (Promega) following manufacturer's instructions.

## Total RNA isolation

WT and $MLL^{4Q4092X}$ MSCs were seeded as sparse (5000 cells/cm$^2$) condition and collected 48h after plating, either untreated or at 8 and 24 hours after ATR inhibition (VE-822 treatment) (medchem express # HY-13902/cs-1861). Cells were directly lysed on plates with TRIzol (Thermo Fisher cat. #15596026), and total RNAs were extracted according to the manufacturer's instructions.

## Gene expression analysis

Quantitative real-time PCR analysis was performed with SuperScript III One-Step SYBR Green kit (Invitrogen #11746). Relative gene expression levels were determined using comparative Ct method, normalizing data on endogenous GAPDH expression levels. The oligonucleotides used for gene expression analysis are listed in Table 1.

RNA-seq library preparation

Contaminating genomic DNA was removed by DNase (Qiagen 843 cat. #79254) digestion. RNA quality and concentration were assessed using the 2100 Bioanalyzer (Agilent cat. #G2939BA) and the Qubit fluorometer (Thermo Fisher cat. # Q33226), respectively. 3'-RNA-seq libraries were prepared by using the QuantSeq 3' mRNA-Seq Library Prep Kit FWD for Illumina (Lexogen cat. #015.24) and starting from 500 ng of total RNA. Libraries were sequenced as single reads of 50 bp with the Illumina HiSeq2500. Three independent biological replicates were performed for each cell line and time point and sequenced as independent libraries.

Differential Gene expression analysis

Raw reads from fastq files were first checked for their quality using FastQC and trimmed using Trimmomatic (Bolger et al., 2014). Trimmed reads were aligned to the reference human genome assembly hg38/GRCh38 using STAR with default parameters. Resulting bam files were converted to bed format by using bedtools with the command "bamtobed". For annotation of the reads to the genome, HOMER was employed with the following command 'analyzerepeats.pl rna hg38 - count 3utr -rpkm'. Genes were considered expressed with rpkm > 1 and used for subsequent analysis. Differential expression analysis was performed using DESeq2 within the HOMER environment.

Computational Analysis of Gene Expression Data

Correlation heatmaps and trajectories of gene expression data were performed by using Clust and visualized using the Multi Experiment Viewer (MeV). Volcano Plots were performed within the R environment.

Gene Ontology (GO) term analysis

Differentially expressed genes in wild-type (WT) compared to MLL$^{4Q4092X}$ MSCs and identified gene clusters from this study and publicly available gene lists (Zanconato et al., 2015) (Provenzano et al., 2009) were used as input for GO term and Reactome pathway analysis with EnrichR. Results were plotted using GraphPad PRISM.

Medaka Fish

The Cab-strain of wt medaka fish (Oryzias latipes) was maintained following standard conditions (i.e., 12 h/12 h dark/light conditions at 27 °C). Embryos were staged according to the method proposed by Iwamatsu. All studies on fish were conducted in strict accordance with the institutional guidelines for animal research and approved by the Italian Ministry of Health; Department of Public Health, Animal Health, Nutrition, and Food Safety.

Kmt2d morpholino injections and drug treatments in Medaka Fish

The available medaka olKmt2d (ENSORLT00000009505.1) genomic sequences were retrieved from a public database (UCSC Genome Browser, http://genome.ucsc.edu/) from human KMT2D (NM_003482.3) transcript. A morpholino (Mo; Gene Tools LLC, Oregon, USA) was designed against the ATG initiation codon within the 5' untranslated region of the medaka ortholog of the (MO-Kmt2d: 5'-CCCTGCTGCTGCTTTGATCTTTTTG-3') of the medaka orthologous of the KMT2D gene. The specificity and inhibitory efficiencies of morpholino was determined as previously described (Conte et al., 2015). MO- Kmt2d was injected at 0.015 mM concentration into one blastomere at the one/two-cell stage. Off-target effects of the morpholino injections were excluded by repeated experiments with control morpholino or by co-injection with a p53 morpholino. For the drug treatment, chorions from injected and control embryos were removed with the hatching enzyme at St32. From St34 onward both morphant or control embryos were grown in 0.15 μM VE822 diluted in 1%DMSO, 1X Yamamoto, for 24 6-days. The solution was refreshed every 24h. For the control experiments, the St34 morphant or control embryos were grown in 1X Yamamoto/ 1 %DMSO.

Cartilage and bones Staining in Medaka Fish

Staining for cartilage (Aldan Blue) and bone (Alizarin Red) in fixed embryos was performed according to standard Medaka skeleton phenotyping protocols (SHIGEN - SHared Information of GENetic resources https://shigen.nig.ac.jp/medaka/medakabook/index.php). Pictures were taken using the DM6000 microscopy (Leica Microsystems, Wetzlar, Germany). Measurement of both cartilage and bone length was performed using ImageJ.

Inelastic Brillouin Scattering

Brillouin scattering is an inelastic scattering process taking place when photons exchange energy with thermally excited acoustic waves or phonons (Antonacci et al., 2018). This causes a small red or blue frequency shift ($\omega\_b$) of the scattered light corresponding to the emission or absorption of a phonon respectively. This frequency shift, is given by

$\omega\_b = 2n/\lambda \sqrt{(M/\rho)} \sin \llbracket \theta/2 \rrbracket$ , where $\lambda$ is the incident wavelength, $\rho$ and n are the density and the material refractive index of the material, M is the longitudinal elastic modulus and $\theta$ is the scattering angle.

Confocal Brillouin Microscope, Data acquisition and analysis

Brillouin scattering is exploited within the Brillouin microscopy for reconstructing sample's 3D images of mechanical properties in a non-invasive manner. Brillouin microscopy is combined with a confocal imaging set-up and a Virtually Imaged Phased Array (VIPA)-based spectrometer (see methods). The source light is emitted from a CW single longitudinal mode laser at 532 nm wavelength (OXXIUS) and focused onto the sample by an oil immersion objective lens (Olympus UP-lanSApo 100) of adjusted numerical aperture equal to one (NA = 1). The same lens was used to collect the backscattered light, providing a theoretical spacial resolution of 0.3 $\times$0.3$\times$ 1.1 $\mu$m. A 3D rapid sample scanning was realized thanks to a nanometric motorized stage (Prior HLD117IX). Finally, the collected light was focused by a single-mode optical fiber, filtered from the undesired elastic scattered light (Lepert et al., 2016) and delivered to the spectrometer.

The spectrometer consists of a modified solid Fabry Perot etalon with a free spectral range of 30 GHz (VIPA, LightMachinery, OP-6721-3371-2) that provides high (>50%) transmission efficiency thanks to an antireflection coated entrance window that minimizes entrance losses. Generally, in Brillouin Microscopy two or more crossed tandem-mounted VIPAs are used reaching a contrast of 60dB (Antonacci et al., 2015). However, multistaged VIPAs mitigate the output efficiency increasing the acquisition times. The single stage VIPA spectrometer allows for registering signal with a contrast higher than 40dB. This in combination with our filtering strategy paves the way for fast acquisition Brillouin Microscopy imaging systems. For Brillouin imaging, cells were seeded at low density (sparse condition) on a $\mu$-slide 4-well ibiTreat (ibidi) and culture for 24h. After the removal of the medium, cells were washed twice with PBS (Sigma-Aldrich), fixed with 4% PFA (Sigma-Aldrich) for 15 min. at room temperature, washed three times with PBS and then left in PBS for the acquisitions on Brillouin microscope.

During data acquisition, the stage longitudinal step size on the sample was 300 nm, the acquisition time 100 ms and the optical power delivered to the specimen was lower than 10 mW. The inventors acquired Stokes and Anti-Stokes lines and fitted them with a sum of Lorentzian functions: the maps of Brillouin shifts reported are the center of Stokes and Antistokes fitted Lorentzian functions. For the analysis, the inventors applied two masks to maps: one on portions of the cells having a shift higher than 7.55 GHz (well representing the overall cell area), the other one on parts having shift higher than 7.75 GHz (representing only stiffer areas). The data reported as bar graphs show the percentage of stiffer parts over the total cell area. All data analysis has been performed using custom-made programs in Matlab.

Statistical analysis

The quantitative data are shown as means plus s.e.m. or as boxplot showing the median and the 10-90th percentile, as specified in each figure legend. No statistical method was used to predetermine sample size and all experiments were repeated at least three times with specific sample sizes reported in each figure legend. Statistical P values calculated by two-tail unpaired Student's t-test are indicated in figures and relative figure legends (where not differentially specified, *P<0.05, **P<0.01, ***P<0.001, ****P<0.00001). P values <0.05 were considered significant. Data collection and analyses of all studies involving animals were conducted randomly and not blinding.

**Results**

Insertion of truncating mutation of KMT2D by CRISPR/Cas9 recapitulates MLL4 LoF ("Loss of Function") affecting KS patients

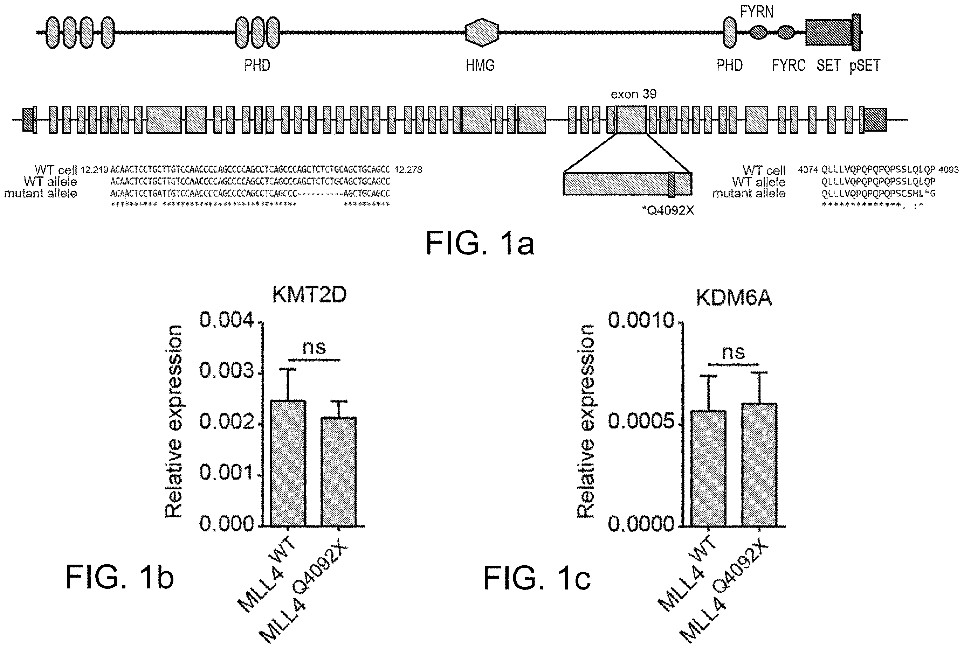

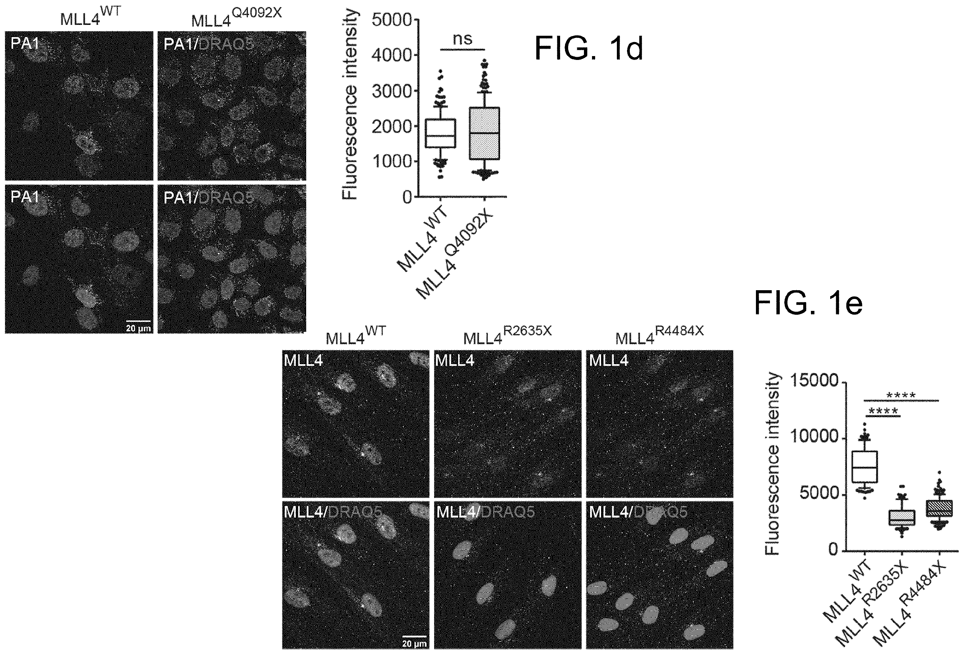

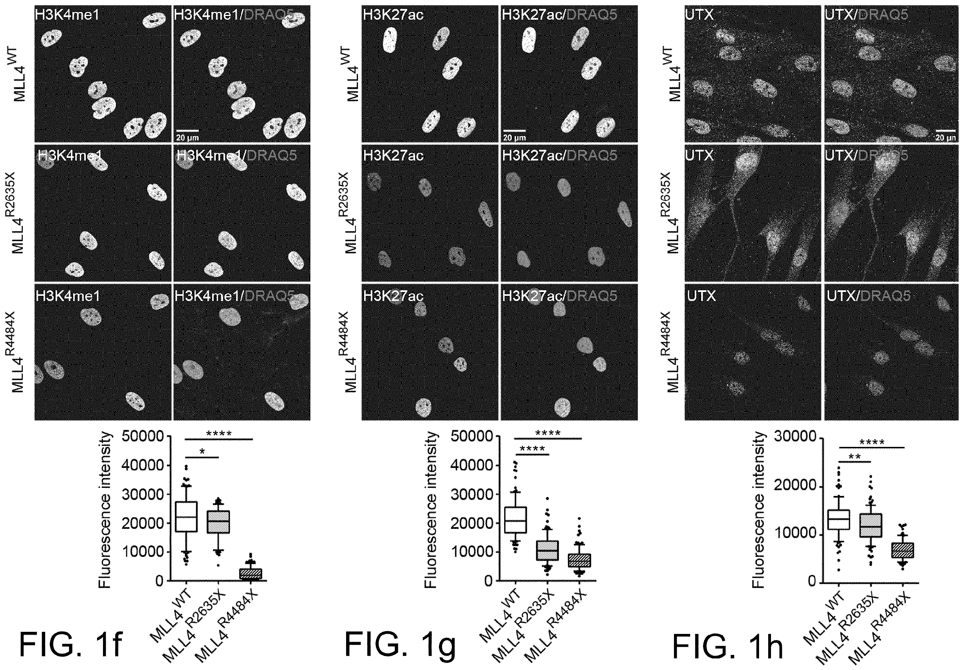

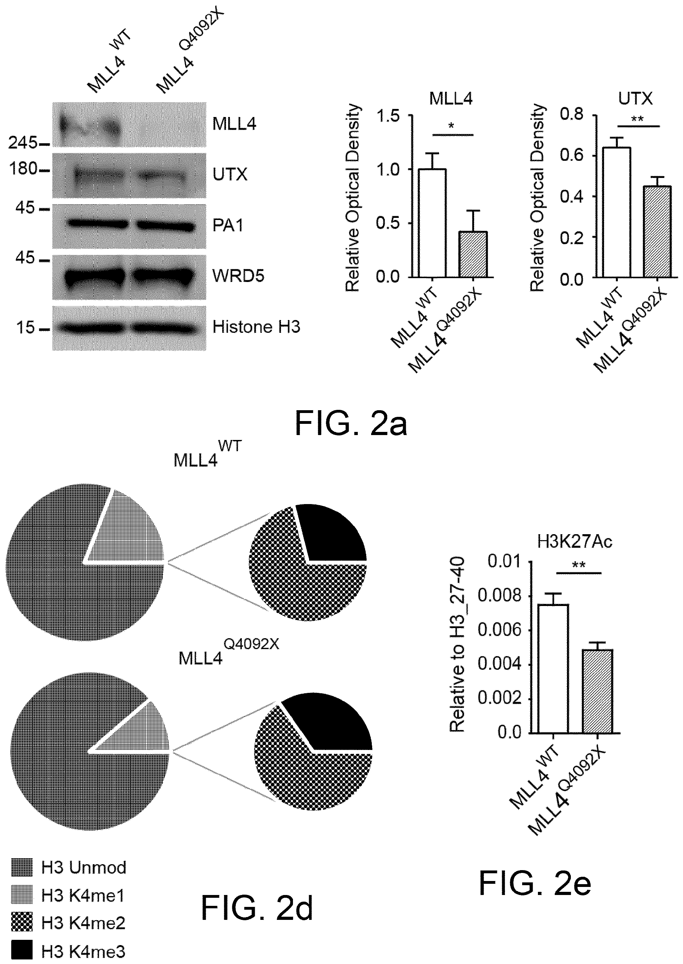

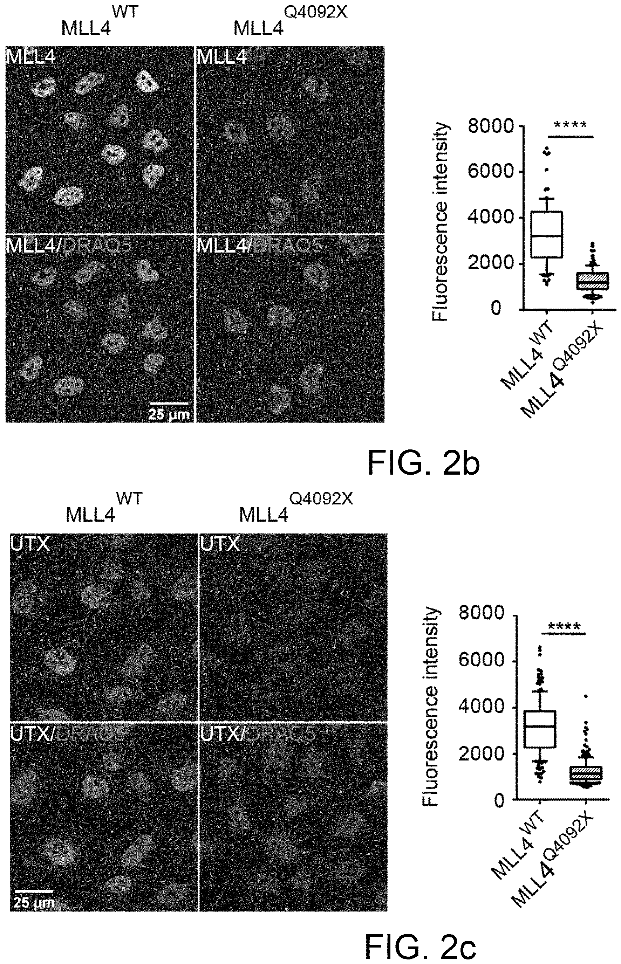

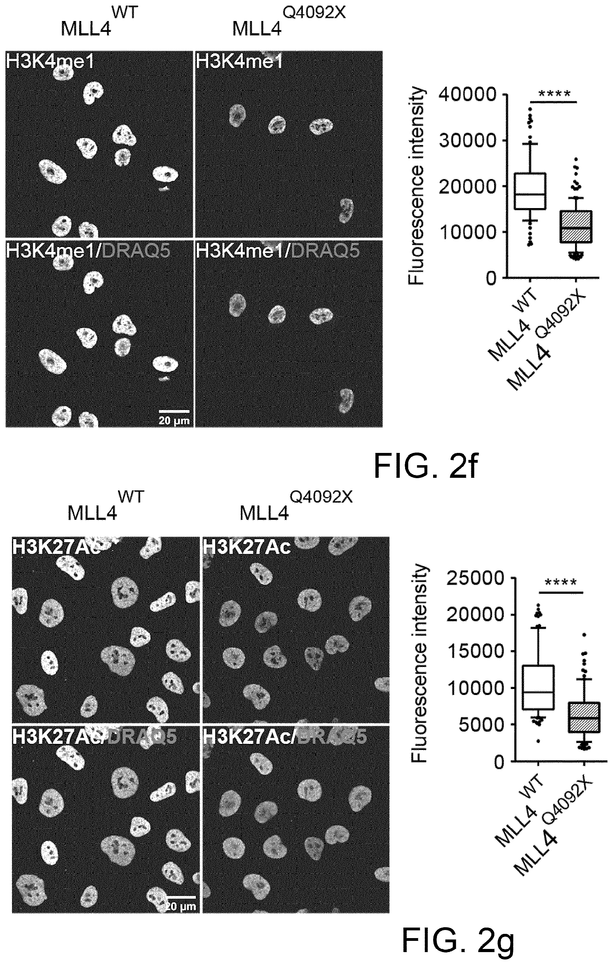

Although it has been established that truncating mutations of *KMT2D* represent the most frequent genetic cause of Kabuki Syndrome (Micale et al., 2014), the consequences of MLL4 LoF have not been defined yet. To address this point, the inventors mimicked the occurrence of truncating mutations in KS patients by inserting a frameshift in the coding region (ex39) of *KMT2D* by CRISPR/Cas9 in mesenchymal stem cells (MSCs). Using this strategy, the inventors derived MSCs carrying a frameshift mutation in heterozygosity, which leads to truncation of MLL4 protein (thereafter named MLL4$^{Q4092X}$) (Fig. 1a). Despite the transcript abundance was unaltered (Fig. 1b), immunoblot analyses showed that the introduced frameshift mutation caused reduction in MLL4 protein level (Fig. 2a), in agreement with previous findings (Micale et al., 2014). Of importance, the relative abundance of the components of the COMPASS-like complex such as WDR5, and PA1 were unaffected by *KMT2D* mutation (Fig. 2a). Of note, UTX protein level resulted diminished in MLL4$^{Q4092X}$ respect to the WT MSCs, indicating that its protein stability is influenced by MLL4 integrity (Fig. 2a and Fig. 1c). Single-cell analyses by quantitative immunofluorescence showed that both MLL4 and UTX levels were uniformly reduced in MSCs carrying MLL4$^{Q4092X}$, while PA1 was unchanged (Fig. 2b-c and Fig. 1d).

Considering that MLL4 possesses a specific mono-methyl transferase activity towards Histone H3, the inventors determined whether its haploinsufficiency could impact on the global level of H3K4me1. The inventors profiled by mass spectrometry the relative changes in histone modifications from nucleosomes purified from WT and MLL4$^{Q4092X}$ MSCs. This analysis showed that H3K4me1 level was relatively lower in MSCs carrying MLL4$^{Q4092X}$ respect to wild-type cells, while H3K4me2/me3 levels were unchanged (Fig. 2d). As MLL4 complex associates with the histone acetyltransferase P300, favoring its recruitment on enhancers (Wang et al., 2017), the inventors also quantified the relative abundance of H3K27 acetylation. Mass spectrometry analysis showed that H3K27Ac is reduced in MLL4$^{Q4092X}$ MSCs compared to WT MSCs. (Fig. 2e). These results were further supported by immunofluorescence analyses (Fig. 2f-g). To ensure that these alterations were not dependent on the developed *in vitro* model for KS, the inventors also measured the same alterations in primary fibroblasts isolated from KS patients (Fig. 1e-h). The obtained data showed that independently from the mutations causing *KMT2D* haploinsufficiency, the diminished abundance of MLL4 resulted in decrease of H3K4me1 and H3K27Ac levels, which was mirrored by a reduction of UTX abundance (Fig. 1e-h). Together, these data showed that truncating mutations of *KMT2D* caused LoF of MLL4 resulting in the impairment of H3K4me1 deposition and a decrease of H3K27Ac level.

## MLL4 favors compartmentalization of transcriptional cofactors

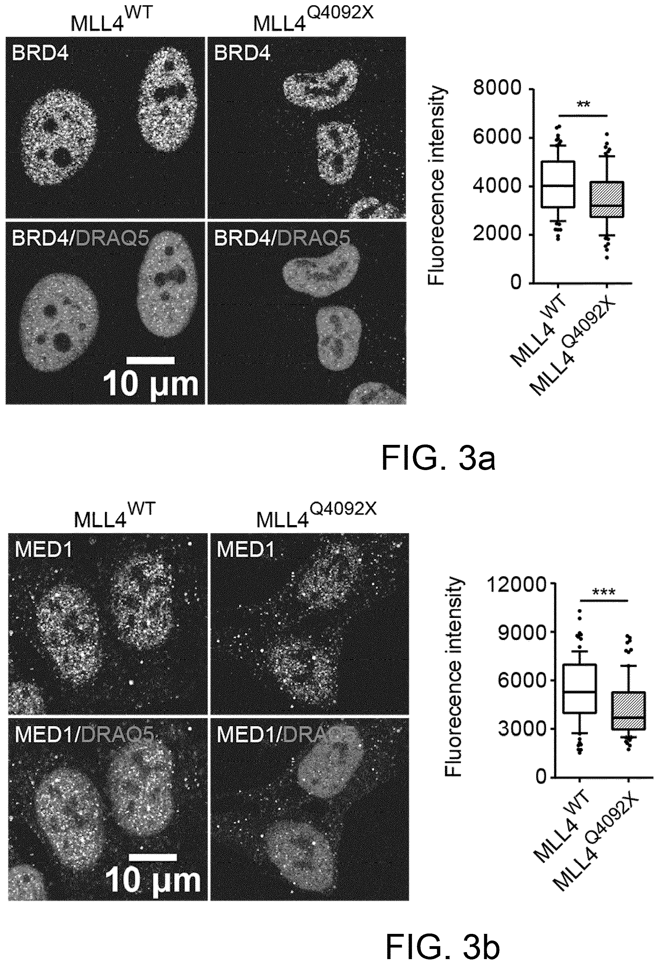

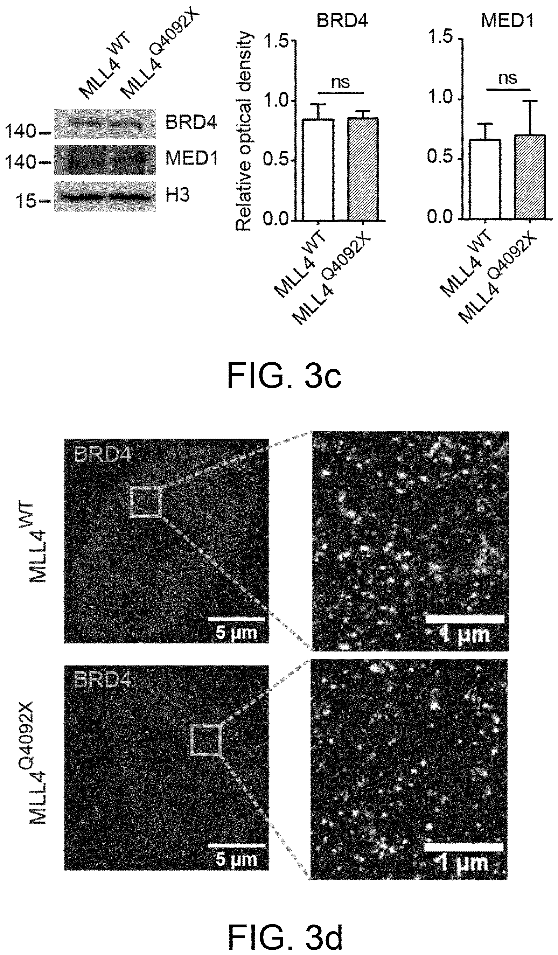

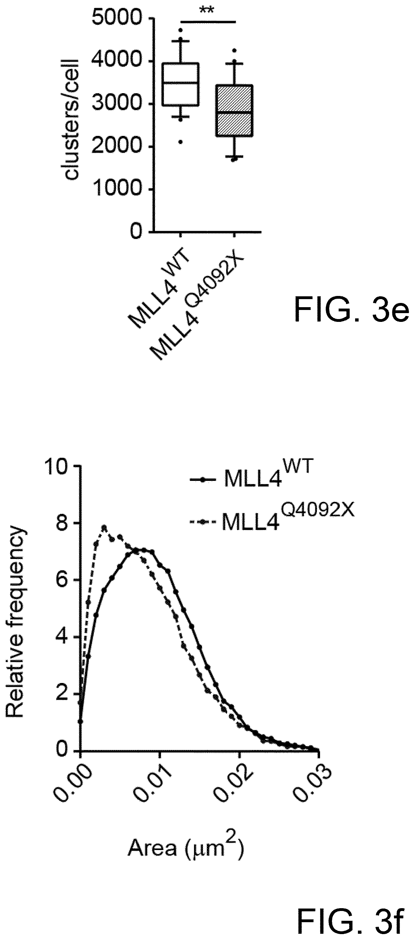

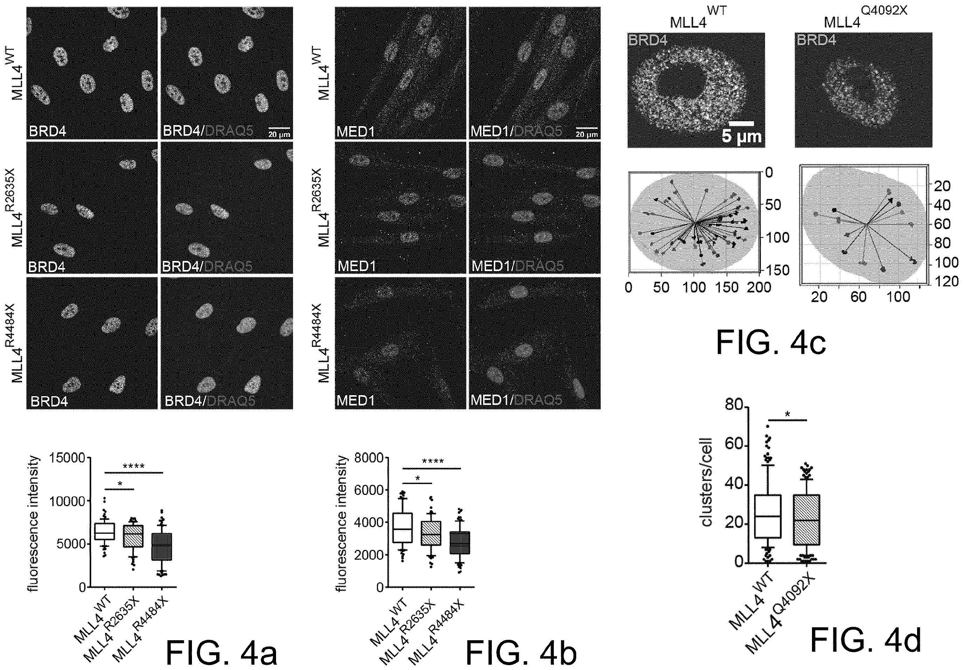

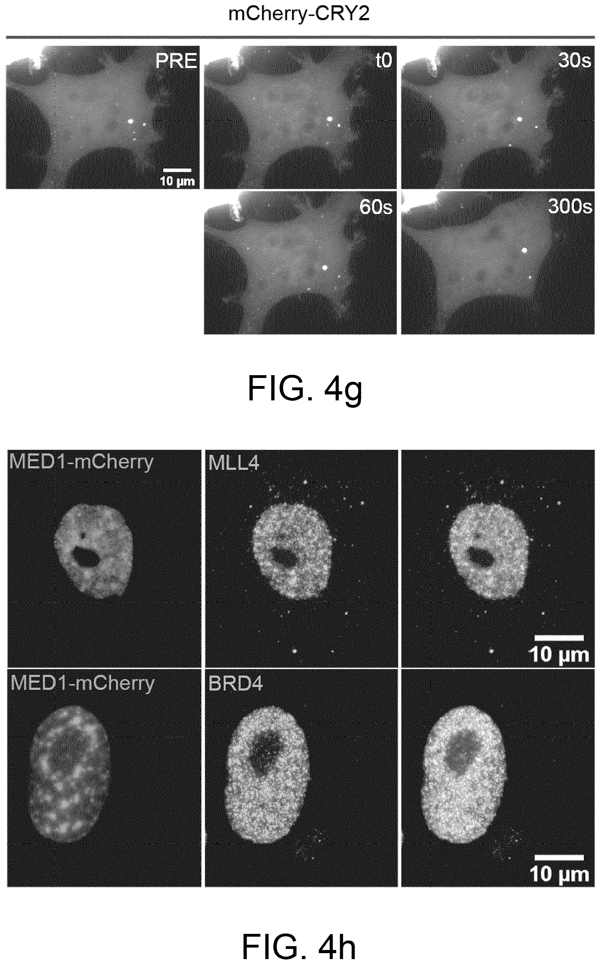

The inventors next investigated whether MLL4 may act in establishing a chromatin context which favors the recruitment of cofactors involved in enhancer activation, including MED1 and BRD4. Therefore, the inventors analysed the distributions of these cofactors, which are organized in biomolecular condensates (Sabari et al., 2018) (Cho et al., 2018) (Nair et al., 2019). Immunofluorescence analyses in MSCs showed that these cofactors were distributed in clusters within the nuclear space (Fig. 3a-b), in line with previous findings in ESCs (Sabari et al., 2018) (Cho et al., 2018). However, the inventors observed that the intensity of BRD4 and MED1 condensates was diminished in MLL4$^{Q4092X}$ respect to the WT MSCs, despite their protein levels were unaltered (Fig. 3a-c). Of importance, the same pattern was also measured in KS patient-derived fibroblasts (Fig. 4a-b). The inventors further measured the number and distribution of BRD4 clusters within the nuclear volume of WT and MLL4$^{Q4092X}$ MSCs (Gregoretti et al., 2016). This analysis showed alteration in the abundance of the condensates in MLL4$^{Q4092X}$ MSCs, whereas their distribution was unaltered (Fig. 4c-d). To better define whether the MLL4 LoF altered the organization of the cofactor condensates, the inventors performed super-resolution imaging by stochastic optical reconstruction microscopy (STORM) on MSCs. STORM revealed that the cofactor BRD4 is distributed throughout the nucleus in clusters ranging from ~0.0051$\mu$m$^2$ to 0.013 $\mu$m$^2$ (Fig. 3d-f). These results suggested that BRD4 molecules were not randomly distributed but rather organized in dense condensates. Of importance, MLL4 LoF perturbed the clustering of BRD4, which formed less condensates, respect to WT MSCs (Fig. 3e). By quantifying the area of the identified clusters, the inventors determined that the size of BRD4 condensates were larger in WT respect to MLL4$^{Q4092X}$ MSCs, ranging from ~0.0039$\mu$m$^2$ to 0.011 $\mu$m$^2$ (Fig. 3f). These results suggested that MLL4 favored the assembly of chromatin clusters enriched for transcription cofactors.

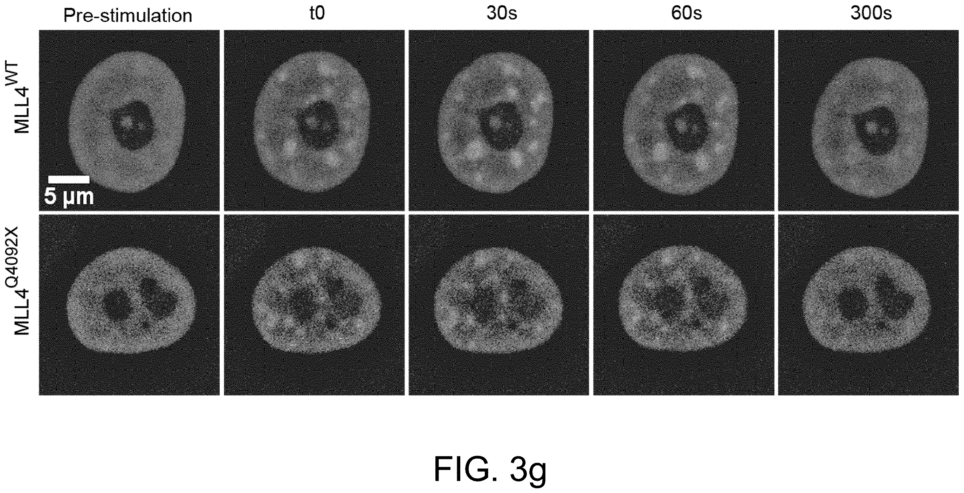

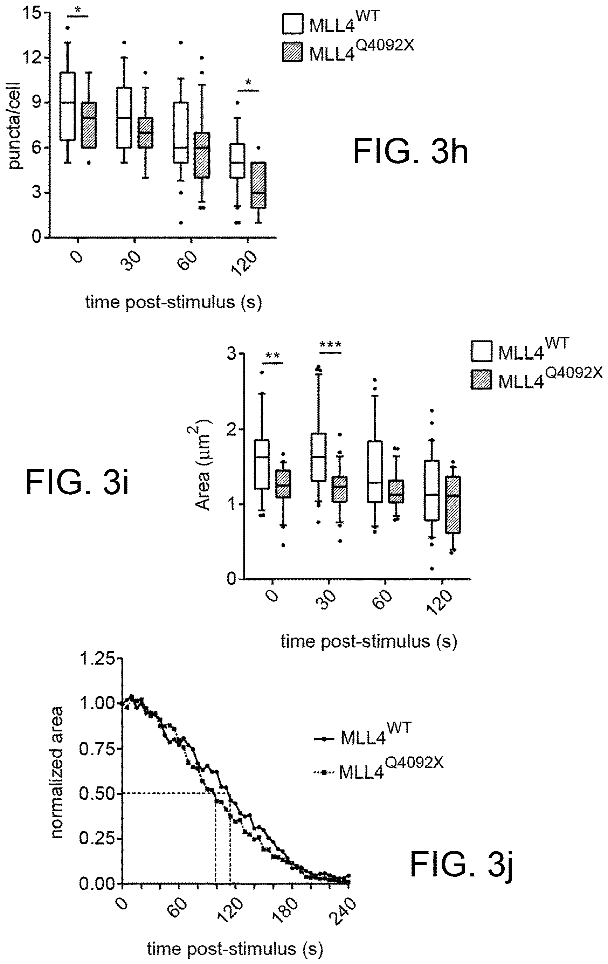

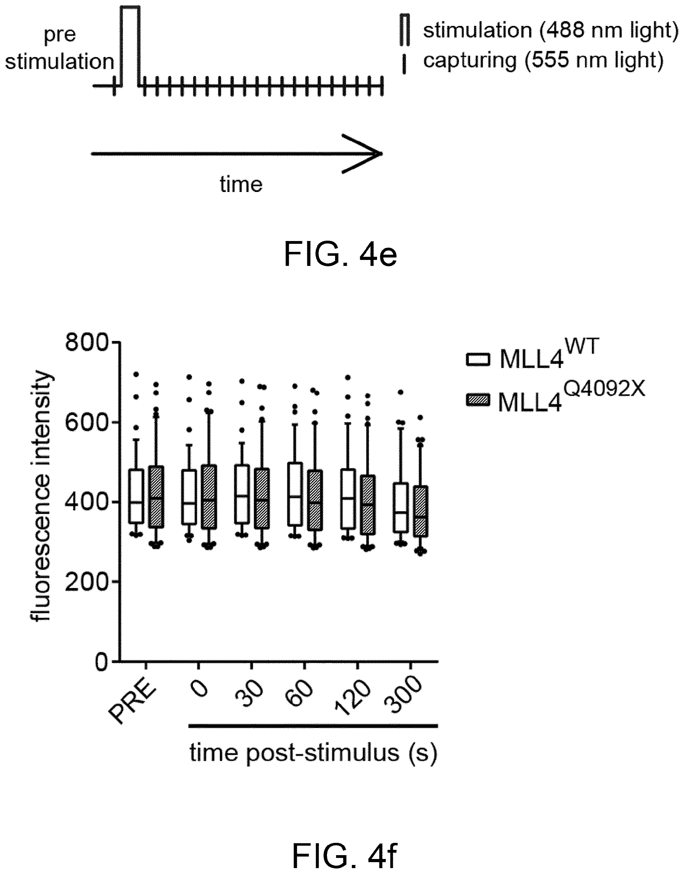

To strengthen these findings, the inventors measured whether MLL4 LoF 4 affected the clustering dynamics of transcriptional condensates within MSC nuclei. To this end, the inventors adopted the optogenetic approach that allows to modulate clustering of proteins containing self-associating IDRs in living cells (Sabari et al., 2018) (Shin et al., 2017). By combining the light-responsive photolyase homology region of Cry2, a domain which self-associates in response to blue light stimulation, with the IDR region of MED1, the inventors followed by live imaging the dynamic formation and disassembly of MED1 clusters. (Fig. 4e). By measuring the light-induced clustering of MED1- IDR (Shin et al., 2017), the inventors observed that a single pulse of blue light was sufficient to drive MED1 assembling in MSCs (Fig. 3g). Time-lapse imaging showed that MED1-IDR clusters were immediately formed after the stimulus, which dissolved within five minutes. Of importance, during the time of acquisition the average intensity of MED1-IDR was unchanged, indicating that the formation and dissolution of clusters were not caused by variation in protein stability, but rather solely depended on the local MED1-IDR concentration (Fig. 4f-g). The inventors further confirmed that the formed puncta corresponded to transcriptional condensates by IF analyses, showing the co-localization with BRD4 and MLL4 (Fig. 4h). Quantitative analyses showed that although MED1-IDR puncta formed with the same timing in WT and MLL4$^{Q4092X}$ MSCs, the total number of formed clusters was reduced in MLL4 LoF condition (Fig. 3h). In addition, the size of the nucleated MED1-IDR clusters was also affected by LoF of MLL4, as the inventors determined a mean area of 1.47$\mu$m$^2$ and 1.15 $\mu$m$^2$ in WT and MLL4$^{Q1012X}$ MSCs, respectively (Fig. 3i). Of importance, WT cells formed transient condensates with a mean lifetime of 119 seconds while clusters in MLL4$^{Q4092X}$ MSCs dissembled at earlier time points, with a lifetime of 102 seconds (Fig. 3j). These results showed that although LoF of MLL4 did not abrogate MED1 clustering, it reduced the nucleation efficacy and the dynamics of assembly and disassembly of transcriptional condensates.

MLL4 forms condensates through liquid-liquid phase separation

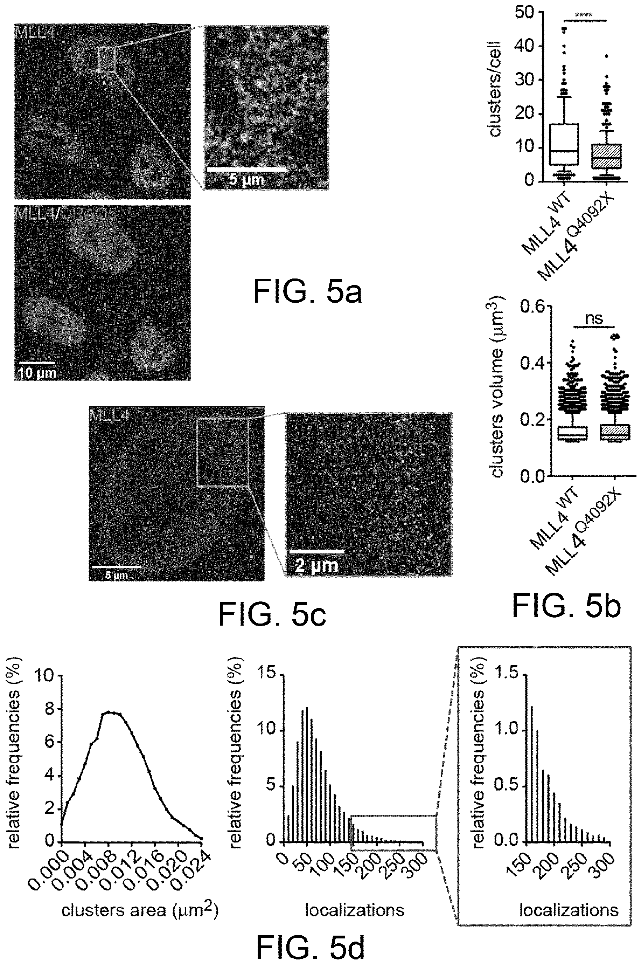

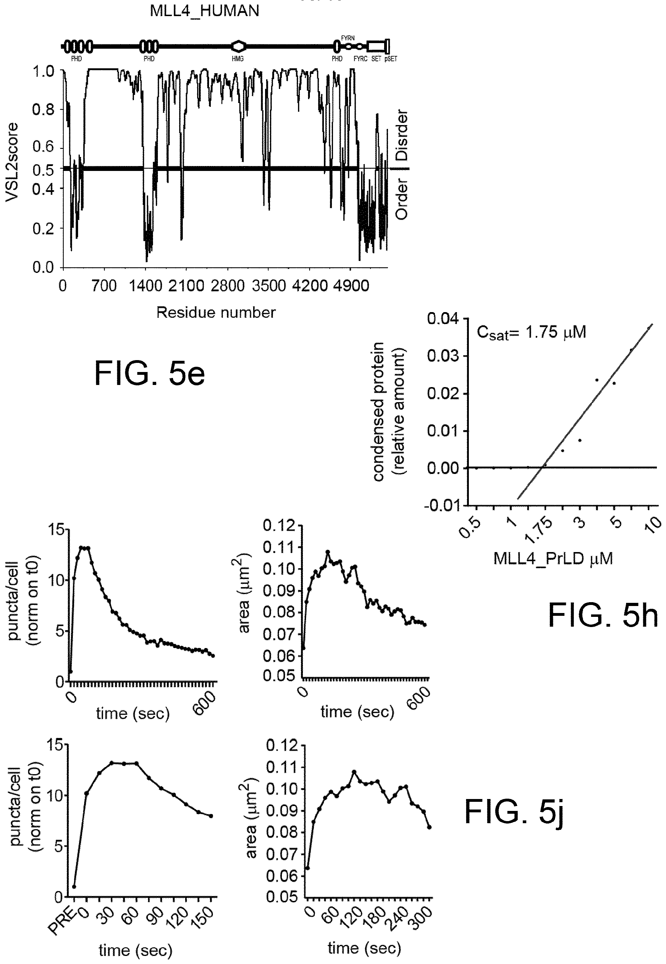

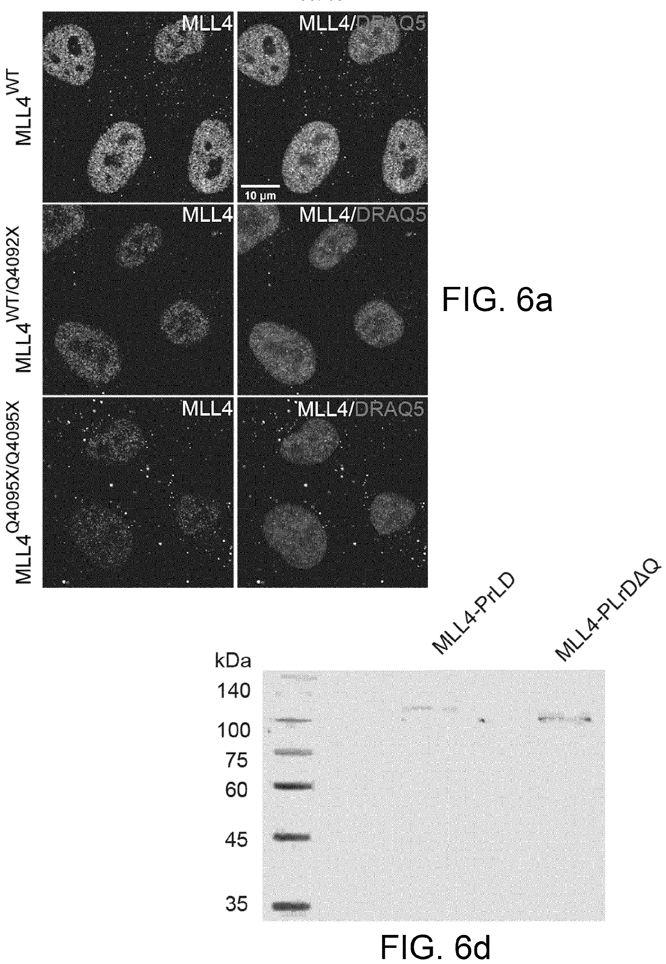

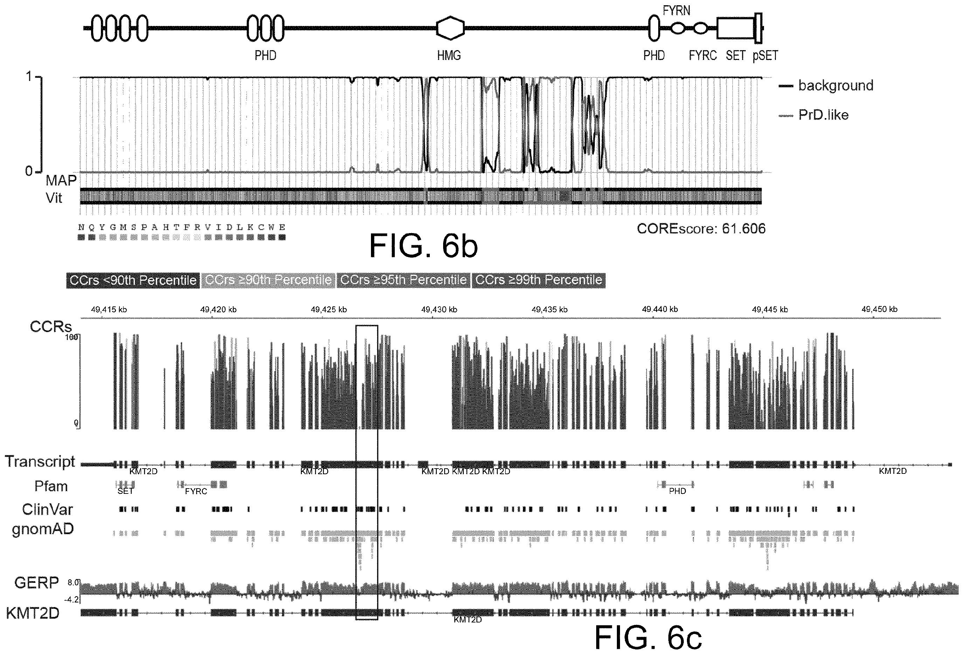

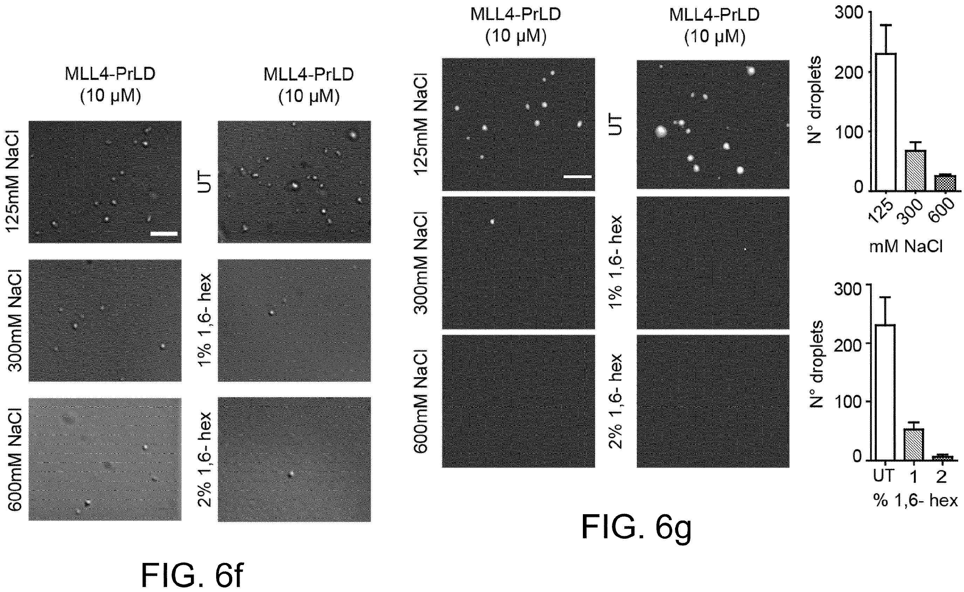

To define the possible mechanism by which the MLL4 COMPASS-like complex supported the clustering of transcriptional condensates, the inventors investigated the distribution of MLL4 protein in the nuclei of MSCs by quantitative imaging. IF analyses showed that MLL4 is organized in puncta, forming clusters that were equally distributed within the nuclear space (Fig. 5a-b). Comparative analyses showed that haploinsufficiency of *KMT2D* determined a reduction of the number of MLL4 condensates, without altering their dimensions (Fig. 5a-b). Of importance, the puncta-like distribution was not due to non-specific signal from the antibody, as the same IF on MLL4 KO MSCs resulted in neither staining signal nor puncta distribution (Fig. 6a). To gain insights about the pattern of MLL4 distribution, the inventors performed super-resolution microscopy by STORM. The obtained results showed that indeed the TrxG component MLL4 exhibited a puncta-like distribution within the nuclear space, with clusters sizing between $0.0062\mu m^2$ and $0.013\mu m^2$ (Fig. 5c-d). Of note, the inventors observed that a small subset of MLL4 protein was clustered in large condensates, each formed by more than 150 localizations, respect to an average distribution of 72.4 localization/cluster. These results suggest that the MLL4 complex is organized in biomolecular condensates which could influence on the distribution of MED1 and BRD4 cofactors.

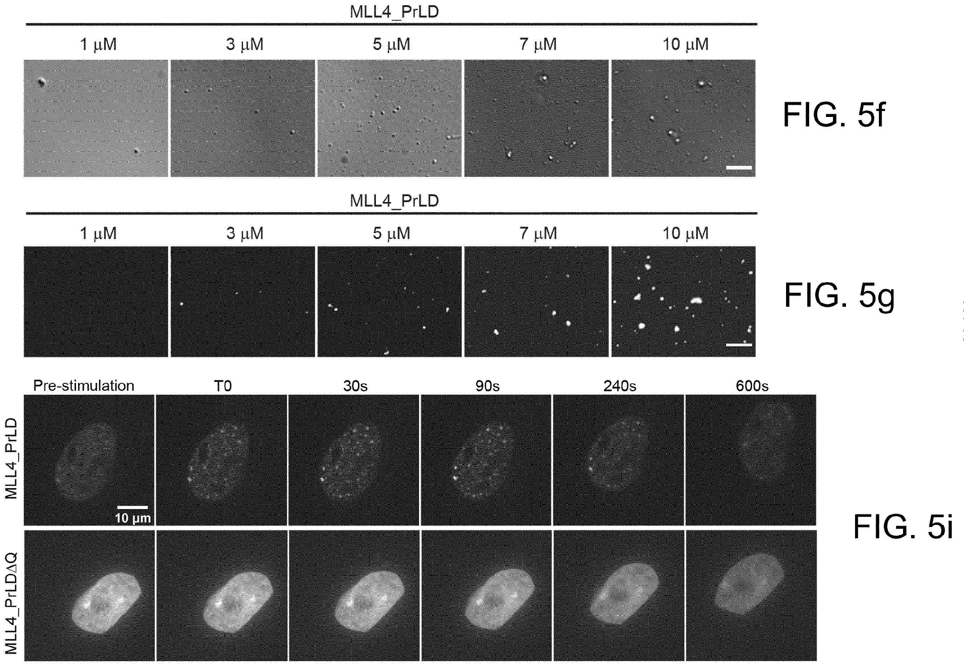

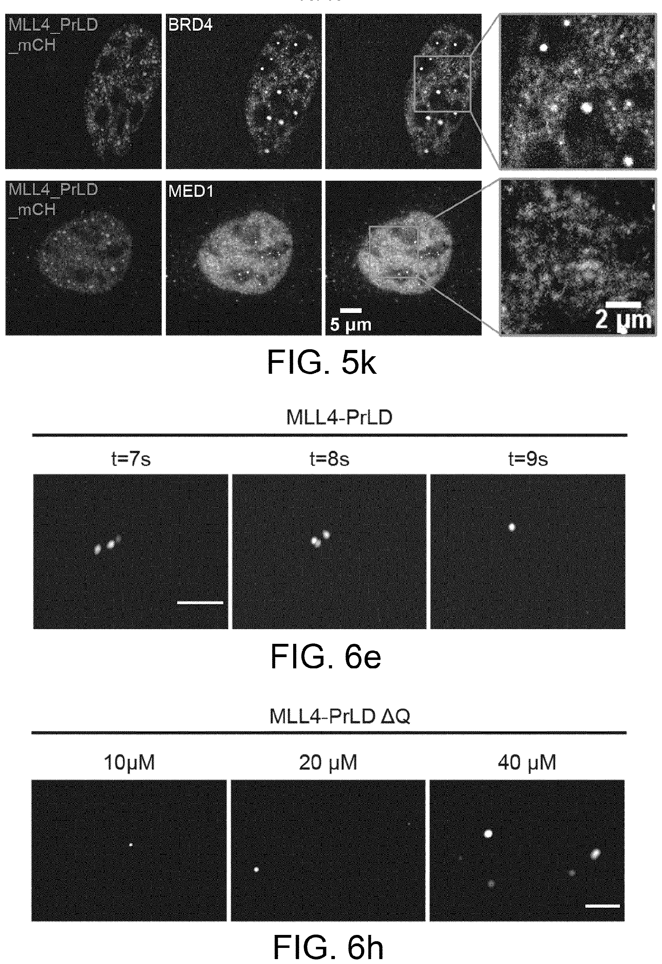

Given that MLL4 function on enhancer activation resulted not uniquely depending on its methyl-transferase activity (Dorighi et al., 2017) (Rickels et al., 2017), the inventors investigated whether it may directly participate in the nucleation of transcriptional condensates. Bioinformatics analyses predicted that MLL4 contains large IDRs that could participate in driving liquid-liquid phase separation (Fig. 5e). In addition, the inventors identified a MLL4-specific prion-like domain (PrLD) enriched of Poly-Gln, which is conserved in multiple species (Fig. 6b-c), and is specifically deleted in KS patients (Lehman et al., 2017) (Cocciadiferro et al., 2018). Given that PrLDs are low complexity regions that promotes multivalent interactions (Alberti, 2017), the inventors determined whether MLL4-IDR harboring the PrLD (MLL4_PrLD [3560]-[4270]) was sufficient to drive liquid-liquid phase separation *in vitro.* The inventors found that purified recombinant mCherry-MLL4_PrLD (Fig. 6d) was able to drive phase separation at physiological ionic strength condition, in the presence of a crowding agent (Fig. 5f-g). Fluorescence microscopy showed rapid formation of spherical droplets that diffused within the liquid volume and occasionally fused (Fig. 6e). To determine the saturation concentration (Csat) at which MLL4_PrLD phase separated, the inventors quantified the intensity of the mCherry signal inside the droplets, respect to the total amount of fluorescent proteins, measured within a range of concentrations. This analysis showed that the Csat of MLL4_PrLD was ~ $1.7\mu M$, behaving similarly to the prototype PrLD-containing protein FUS (Fig. 5g-h) (Wang et al., 2018). Of importance, the phase separation of MLL4_PrLD was reversible as the formed droplets were dissolved by increasing the ionic strength or by competing out the hydrophobic interactions by adding 1,6-hexanediol (Fig. 6f-g). Given that the identified MLL4_PrLD contains a PolyQ stretch, the inventors deleted this region from the mCherry-MLL4_PrLD protein and assessed its contribution to phase separation. The inventors purified the recombinant mCherry-MLL4_PrLDΔQ (Fig. 6d) and measured its capacity to phase separate, *in vitro.* The removal of the polyQ stretch was sufficient to strongly reduce the proficiency of MLL4-PrLD to form droplets, suggesting that this region is required to drive phase separation (Fig. 6h).

The inventors then investigated whether MLL4-PrLD participated in the formation of transcriptional condensates in living cells. To this end, the inventors sought to dynamically modulate the local protein concentration by using light-activated OptoIDR approach (Shin et al., 2017). The inventors tagged the MLL4-PrLD with mCherry and fused it to the Cry2 module that self-associates in response to blue light exposure (Bugaj et al., 2013). The inventors observed that a single pulse of blue light was sufficient to drive clustering in most of the expressing cells, forming spherical droplets (Fig. 5i). Continuous time lapse imaging showed that following light stimulation, the OptoMLL4 nucleated forming droplets whose number and dimensions increased rapidly, reaching a plateau within 60 seconds (Fig. 5j). Of importance, the light-induced clustering of the OptoMLL4 resulted reversible as the droplets persisted for 300 seconds to then diffuse into the nucleoplasm, reaching the initial distribution (Fig. 5i, j). Notably, the deletion of the PolyQ track was sufficient to abrogate nucleation event, as the stimulation of the MLL4_PrLDΔQ did not give rise to detectable droplets (Fig. 5i). Finally, by performing co-localization analyses, the inventors showed that optoMLL4 clustered with the cofactors BRD4 and MED1 (Fig. 5k). Based on these results, the inventors concluded that MLL4 participates in the formation of transcriptional biomolecular condensates through its prion-like domain.

MLL4 LoF unbalances PcG compartments

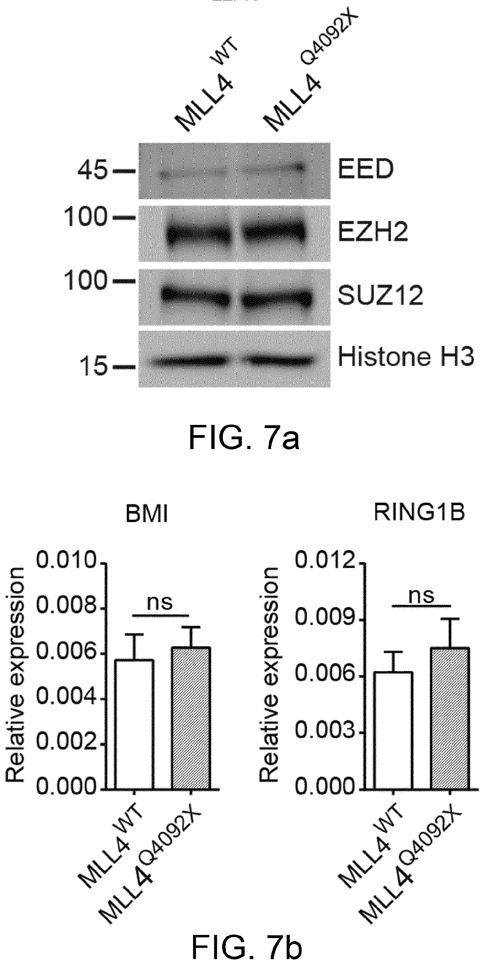

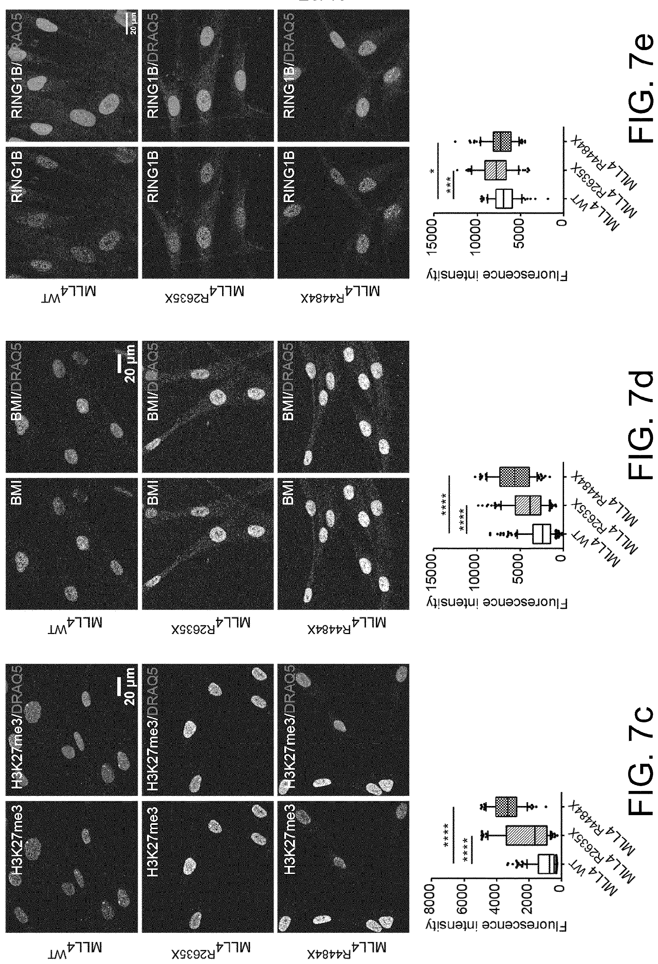

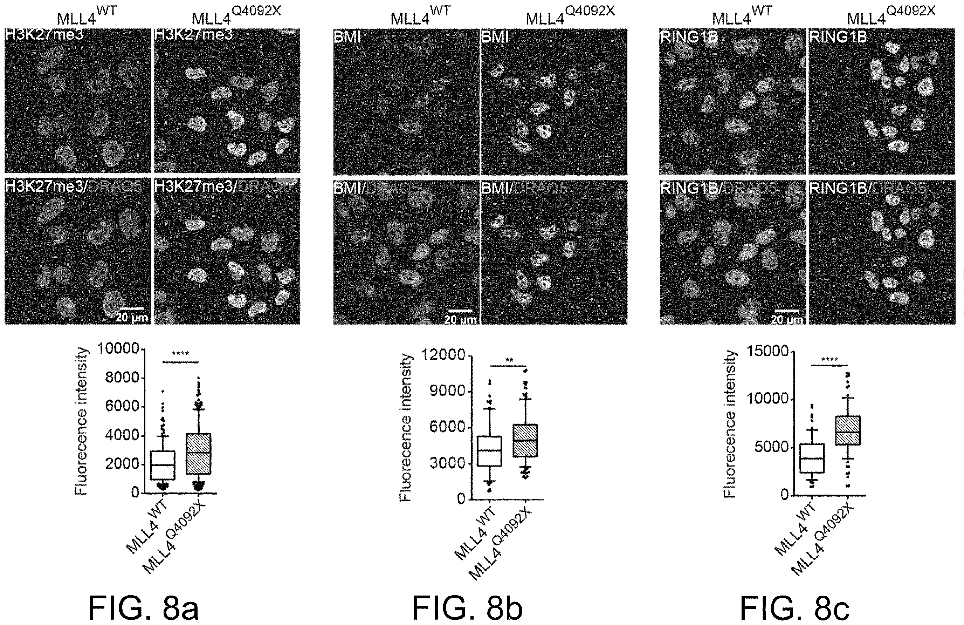

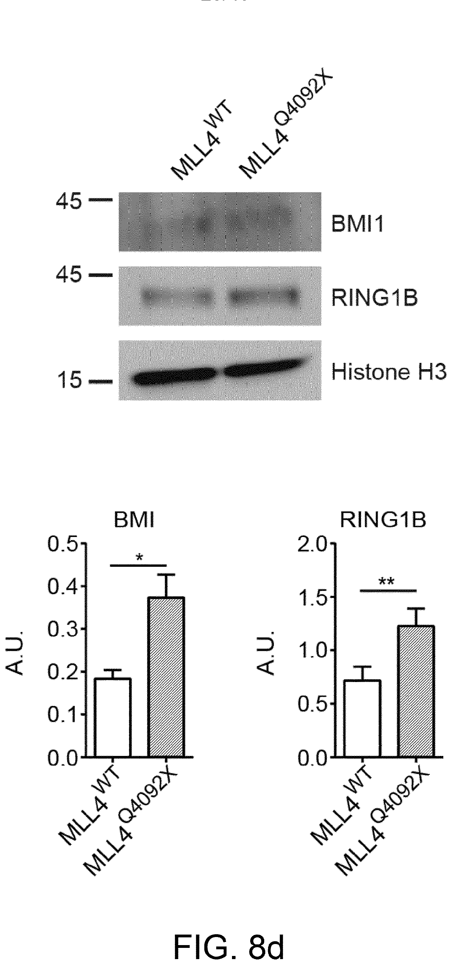

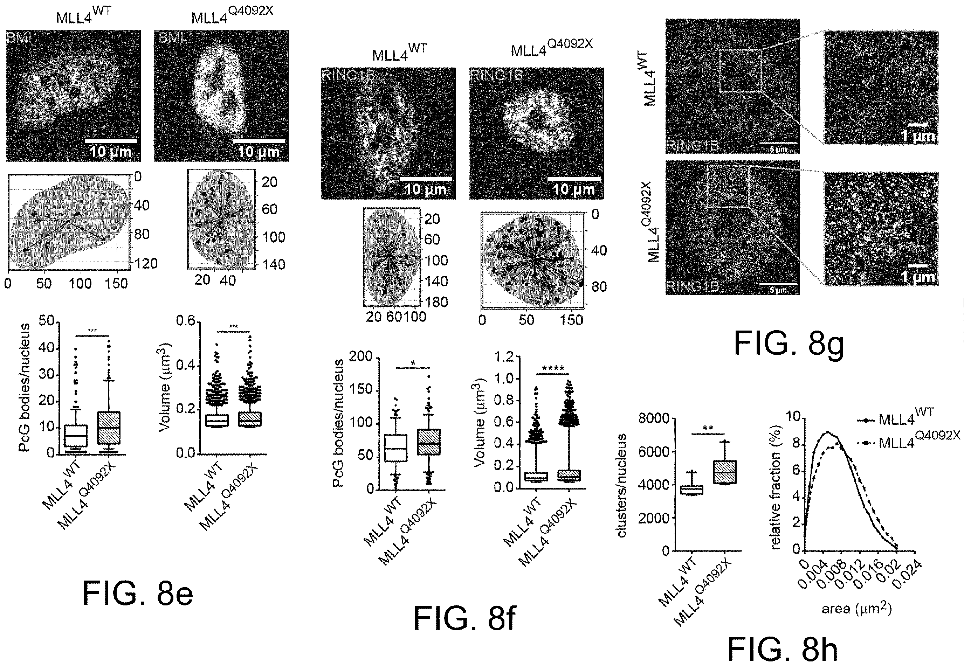

Given the genetic and functional antagonism between TrxG and PcG complexes (Schuettengruber et al, 2017), the inventors investigated whether the perturbation of transcriptional condensates caused by MLL4 LoF could also affect the repressive compartments associated with PcG complexes. By performing quantitative immunofluorescence analyses the inventors found that, although the protein level of PRC2 components (EED, EZH2 and SUZ12) resulted unaltered (Fig. 7a), the deposition of H3K27me3 was increased (Fig. 8a). By analyzing the canonical components of PRC1 complex, the inventors determined an increment of RING1B and BMI1 signals, which resulted distributed in condensates (Fig. 8b-c). By measuring the total quantity of PcG proteins, the inventors detected a higher level of both BMI1 and RING1B, while their transcripts resulted unperturbed (Fig. 8d and Fig. 7b). Of importance, a similar enrichment for H3K27me3 and PRC1 components was also measured in patient-derived fibroblasts (Fig. 7c-e). To determine the spatial organization of PcG foci, the inventors measured the number and distribution of BMI and RING1B clusters within the nuclear volume of WT and MLL4$^{Q4092X}$ MSCs. These analyses showed that MLL4$^{Q4092X}$ MSCs contained a higher number of PcG foci, with a similar distribution within the nuclear volume (Fig. 8e-f). To better quantify the increase of clustering upon LoF of MLL4, the inventors performed nanoscopy imaging of PRC1 components by STORM (Fig. 8g-h). Quantitative analyses determined that RING1B formed heterogeneous clusters, with a mean size of ~0.007 $\mu$m$^2$. Respect to the distribution of the PcG condensates in wild-type cells, the inventors measured an increment of large (> of 0.012 $\mu$m$^2$) clusters within the nuclei of MLL4$^{Q4092X}$ MSCs, respect to the wild-type counterpart (Fig. 8g-h). These data indicated that MLL4 LoF, besides impairing transcriptional condensates, affected repressive compartments with an increase of PcG proteins clustering.

MLL4 regulates nuclear mechanics and chromatin compaction

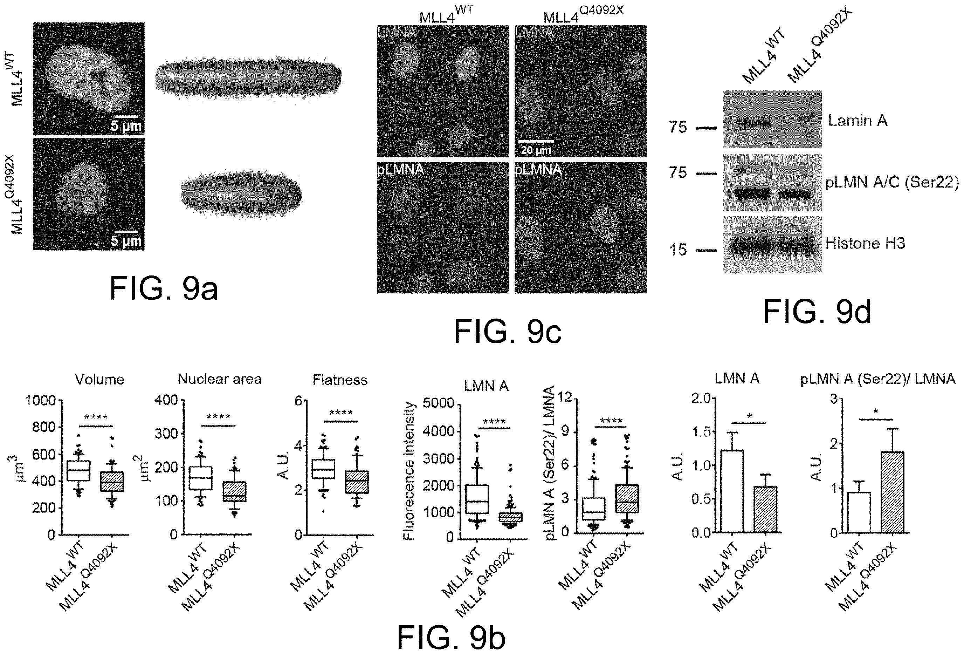

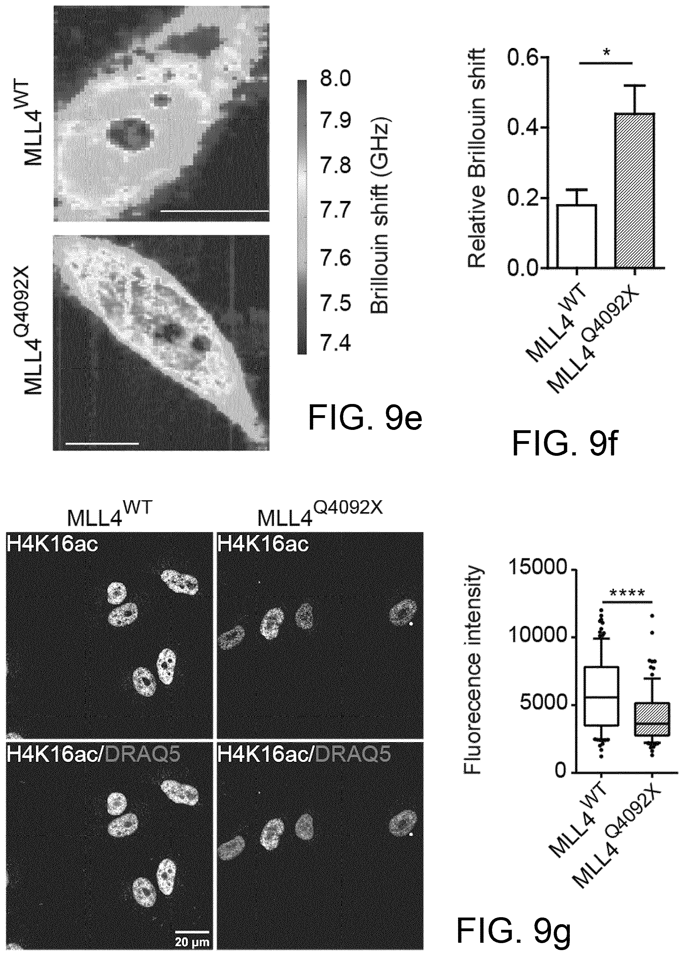

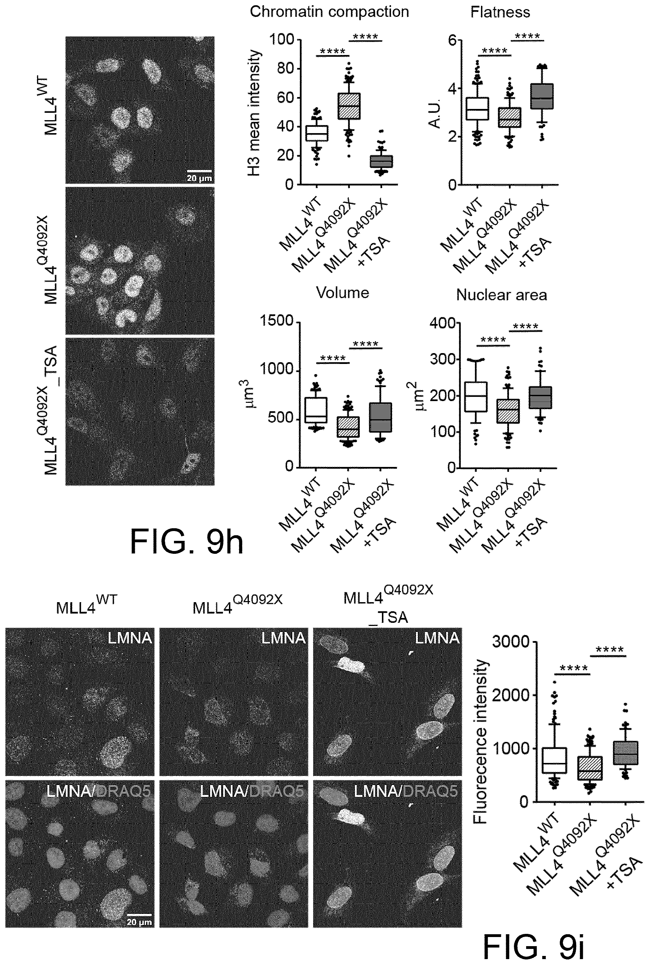

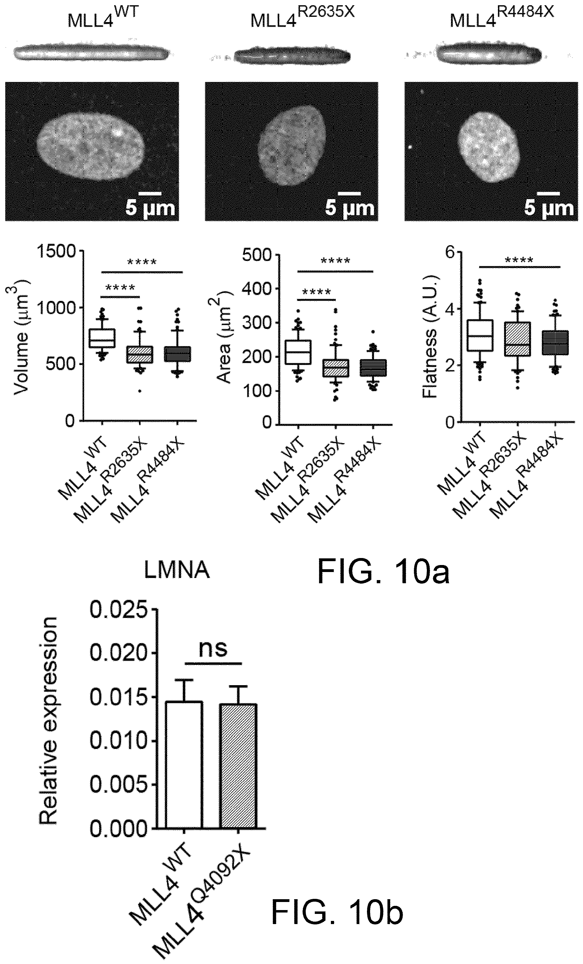

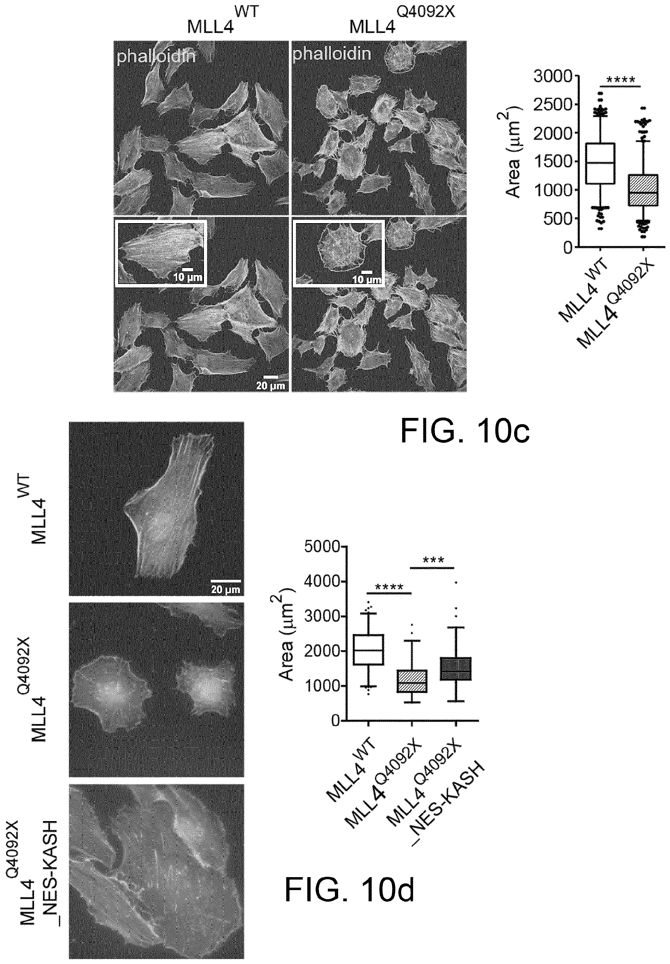

The obtained results supported the notion that LoF of MLL4 is sufficient to alter the balance between enhancer-centered and PcG condensates. Given the physical properties of chromatin that exerts forces that shape 3D genome folding and nuclear structure (Bustin and Misteli, 2016) (Rada-Iglesias et al., 2018), the inventors focused on the possible consequences of altering chromatin compartmentalization by analyzing the effects on nuclear architecture and nuclear mechanics. To this end, the inventors determined the nuclear shape by confocal scanning microscopy and the inventors observed that the nuclear morphology of MLL4$^{Q4092X}$ MSCs was altered, respect to WT cells (Fig. 9a). The inventors measured a consistent reduction of nuclear area and volume of MLL4$^{Q4092X}$ MSCs, which resulted in a decreased of nuclear flattening (Fig. 9b). Of note, these alterations in the nuclear shape were also retrieved in primary fibroblasts isolated from KS patients, indicating that they are not cell-type dependent (Fig. 10a). These findings suggested that, although MSCs were grown sparsely on stiff substrate favoring force transmission to the nuclei (Elosegui-Artola et al., 2017), the MLL4 LoF impaired nuclear architecture. To verify this hypothesis, the inventors tested the relative protein level of Lamin A/C whose abundance is modulated in response to changes in tensile forces (Swift et al., 2013). The inventors found that although the transcript level of Lamin A/C did not change, (Fig. 10b), the relative protein abundance was diminished in MLL4$^{Q4092X}$ MSCs respect to wild-type cells (Fig. 9c-d). Considering that changes in mechanical stresses is balanced by the assembly and protein turnover of Lamin A/C, the inventors determined its phosphorylation state which is coupled with nuclear lamina organization (Buxboim et al., 2014). The inventors observed that by maintaining MSCs in sparse condition, the tensional forces were coupled with high level of stable, low-phosphorylated Lamin A/C (Fig. 9c-d) (Buxboim et al., 2014). Of importance, MLL4 LoF was sufficient to drive an increment of Lamin A/C phosphorylation and protein turnover (Fig. 9c-d), resembling the pattern observed in low nuclear stress condition (Buxboim et al., 2014). To directly determine the nuclear mechanical properties, the inventors performed all-optical, label-free and non-invasive measurements by Brillouin microscopy (Buxboim et al., 2014). With this technique, the highfrequency longitudinal elastic modulus can be determined in intact cells by measuring the Brillouin frequency shift. As a result, the inventors found that the nuclear stiffness of MLL4$^{Q4092X}$ MSCs was increased respect to the WT cells, as indicated by the relative increment in the Brillouin shift (Fig. 9e-f). The inventors then determined whether the retrieved alterations in nuclear architecture were coupled with perturbation in cytoskeleton organization. By measuring the level of actin polymerization, the inventors determined a decrease of the occupied cell area in MLL4$^{Q4092X}$ MSCs, which was linked to reduction of cytoskeleton stress fibers (Fig. 10c). Given that the LINC complex mediates the cell/nucleus mechanical coupling, the inventors determined whether the altered intranuclear forces could be the cause of the perturbed cytoskeleton organization in MSCs. By over-expressing dominant-negative GFP-Nesprin1/2-KASH protein, that interferes with the formation of a functional LINC complex, the inventors observed a partial rescue of the cell size and the proper actin polymerization (Fig. 10d).

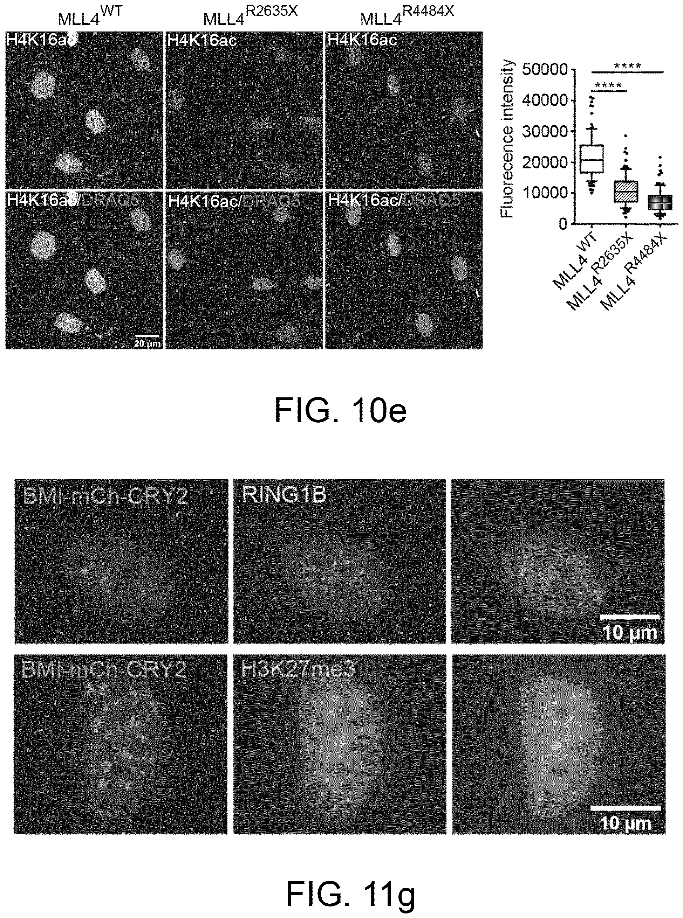

The inventors then investigated whether the mechanical stresses detected in MLL4$^{Q4092X}$ MSCs were associated with changes in chromatin compaction. At first the inventors measured the relative level of H4 lysine 16 acetylation (H4K16ac), which controls chromatin structure by weakening inter-nucleosomal interactions (Shogren-Knaak et al., 2006). IF analyses showed that H4K16Ac was diminished in MLL4 haploinsufficient MSCs (Fig. 9g) and in patient-derived fibroblasts (Fig. 10e). In parallel, the inventors also measured an increment of chromatin compaction which was dependent on the histone acetylation state as treatment with HDAC inhibitor rescued the altered chromatin organization and nuclear architecture (Fig. 9h-i). Specifically, TSA treatment of MLL4$^{Q4092X}$ MSCs was sufficient to re-establish proper nuclear morphology and structure, as shown by the relative abundance of Lamin A/C (Fig. 9h-i). In sum, these findings showed that MLL4 LoF caused nuclear mechanical stress by affecting chromatin organization and nuclear architecture.

Polycomb clustering impinges on nuclear architecture

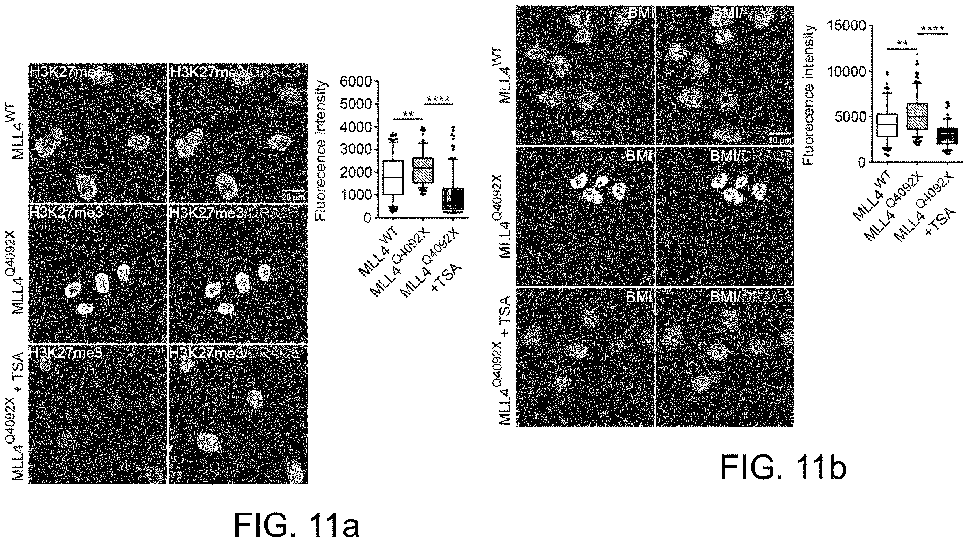

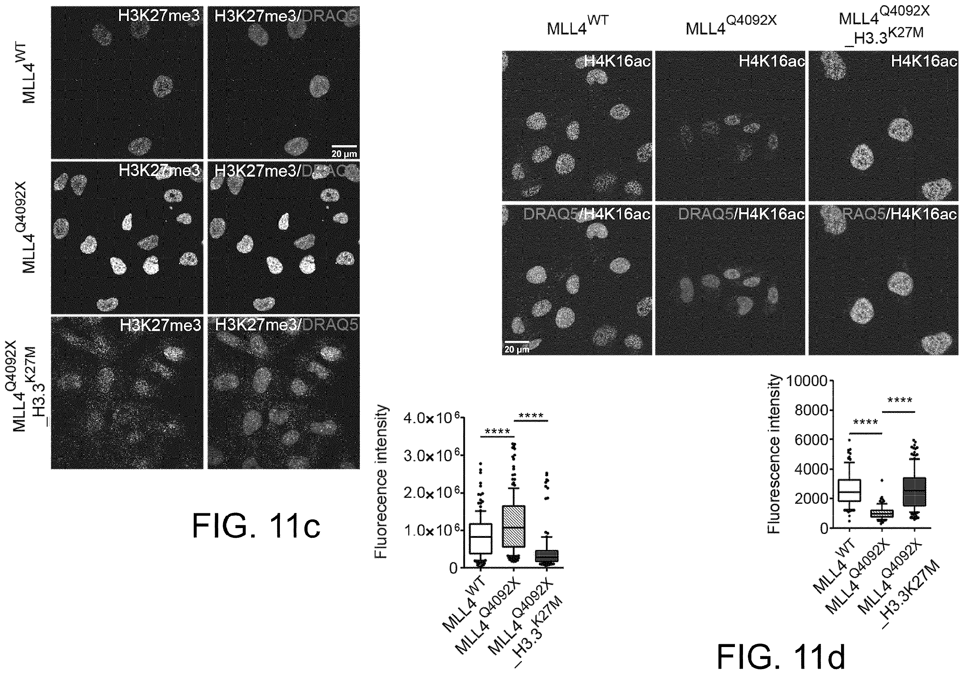

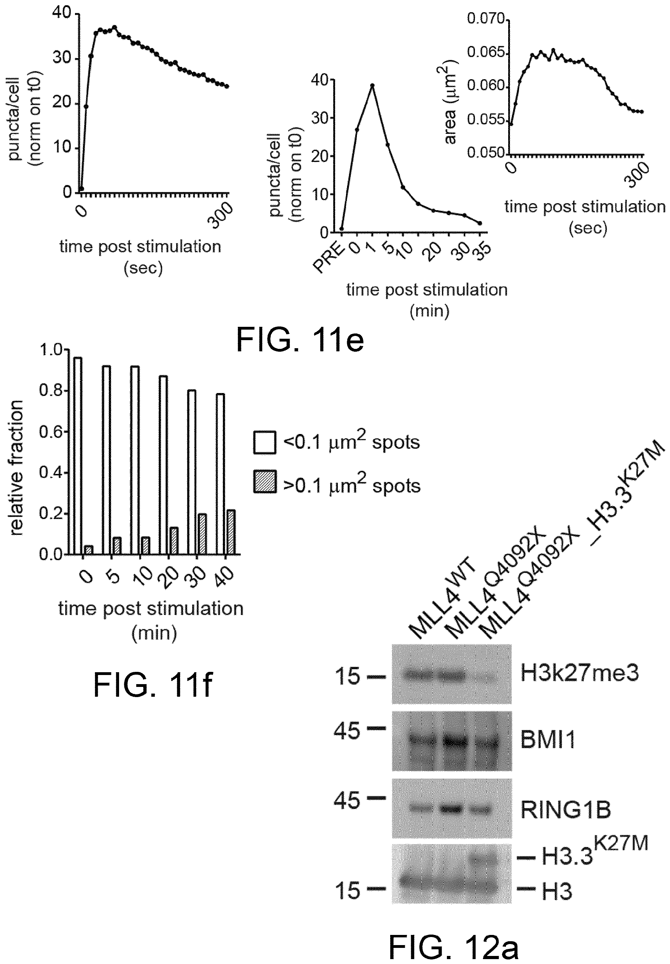

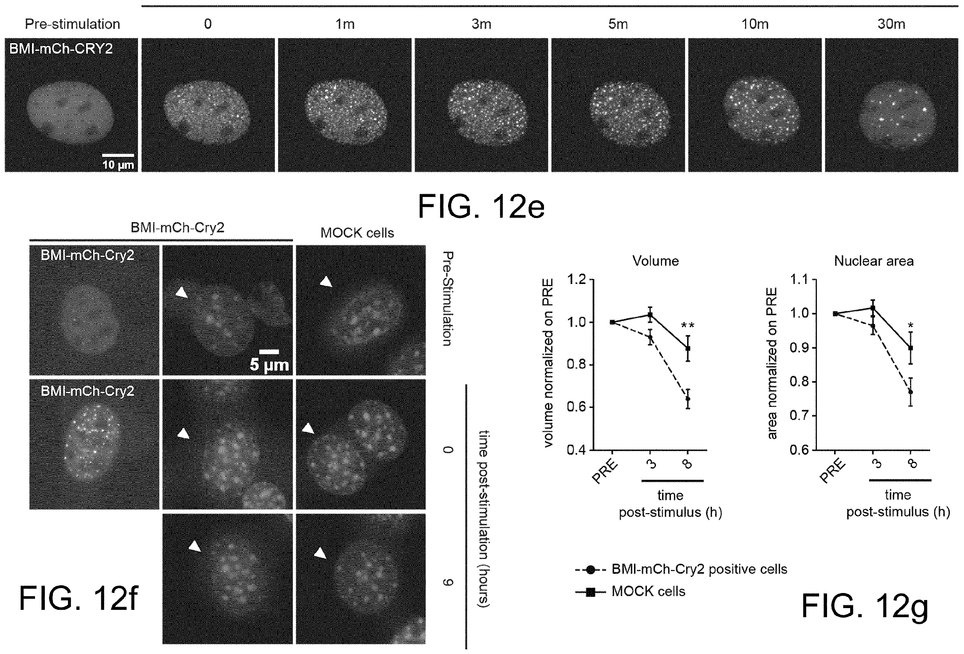

Besides altering nuclear morphology, the inventors noticed that the treatment with HDAC inhibitor reduced the H3K27me3 level and the clustering of PcG condensates in MLL4$^{Q4092X}$ MSCs (Fig. 11a-b). These findings suggested that the relative abundance of Polycomb-associated chromatin state could affect chromatin compartmentalization and nuclear mechanics. To verify this possibility, the inventors rescued the PcG-associated chromatin state by overexpressing in MLL4$^{Q4092X}$ MSCs H3.3 carrying the K27M mutation (H3.3K27M), which has a dominant negative effect on the tri-methylation level of the endogenous Lysine (Lewis et al., 2013). In line with previous findings, the overexpression of H3.3K27M reduced the level of H3K27me3 in MLL4$^{Q4092X}$ MSCs (Fig. 12a and Fig. 11c). Of importance, the inventors also found that in the same setting the protein level of both BMI1 and RING1B was re-established to the same state measured in WT MSCs (Fig. 12a). By analyzing the distribution of Polycomb proteins in the presence of H3.3 K27M the inventors found that both BMI1 and RING1B clusters were strongly reduced.

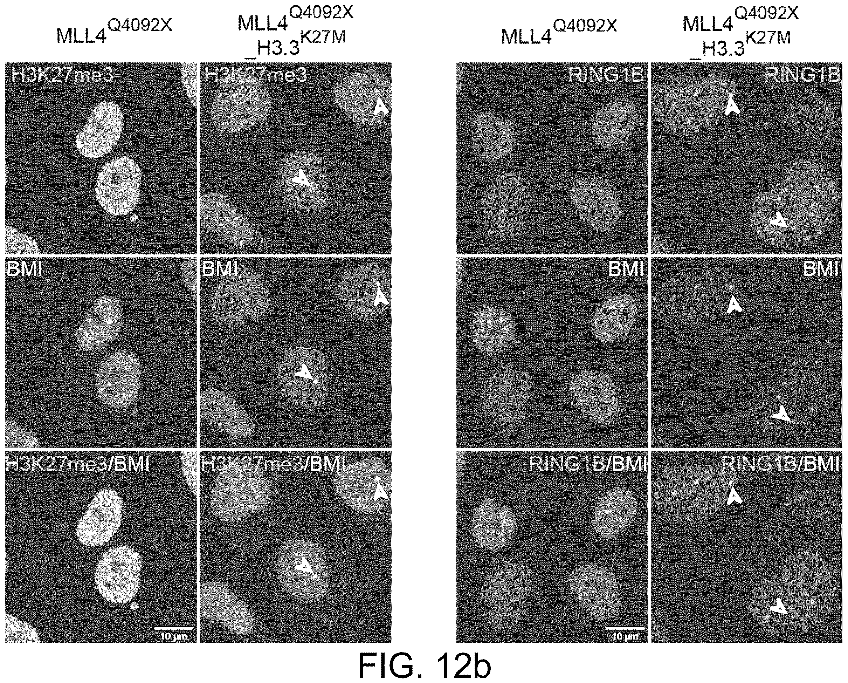

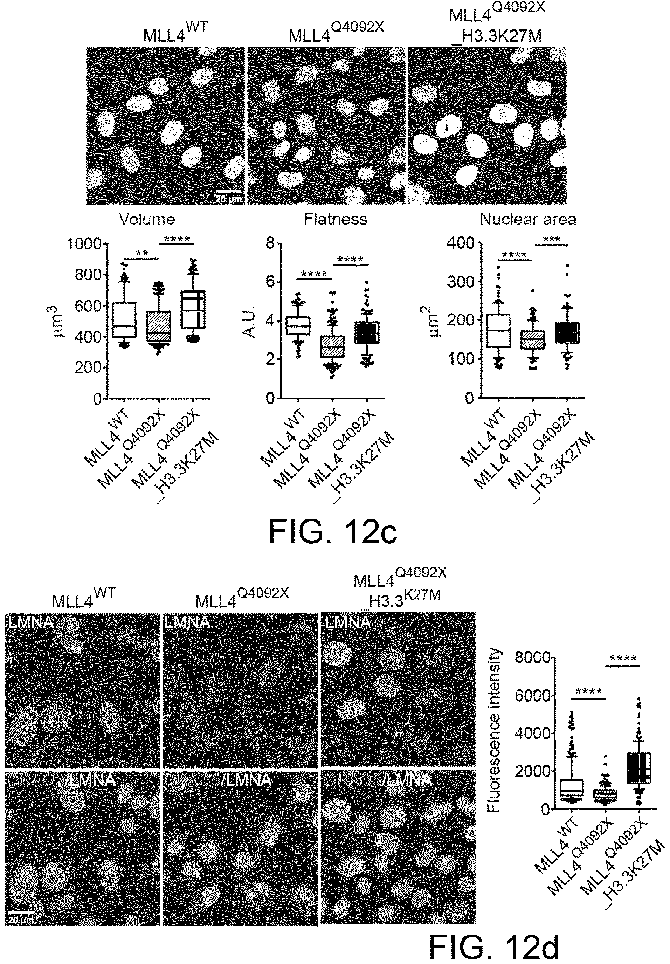

However, the remaining PcG proteins formed also larger clusters in which H3K27me3 signal co-localized with both BMI1 and RING1B proteins (Fig. 12b). Nevertheless, the inventors found that the reduction of PcG activity rescued the nuclear shape as MLL4$^{Q4092X}$ MSCs expressing H3.3- K27M showed an increment of their nuclear volume and area, with a nuclear flattening similar to WT MSCs (Fig. 12c). These changes were coupled with an increment of Lamin A/C protein, re-establishing the nuclear architecture (Fig. 12d). In addition, the rescued PcG levels were also associated with an increment of H4K16ac, suggesting a decrease in chromatin compaction (Fig. 11d).

To verify whether Polycomb-mediated compartmentalization represents a driving force establishing nuclear mechanics, the inventors adopted an optogenetic approach to induce BMI1 clustering in living cells. By measuring the light-induced clustering of BMI1-Cry2, the inventors observed that a single pulse of blue light drove the formation of relative stable BMI1 clusters, with a lifetime of 12 minutes (Fig. 12e, Fig. 11e). The inventors observed that in response to light stimulation the number and size of BMI1 clusters increased to reach a plateau in two minutes, forming heterogeneous clusters. Of note, the light-induced condensates persisted up to 30 minutes post-stimulation, forming large clusters that were enriched at Polycomb binding sites (Fig. 11e-f). Of importance, prolonged light stimulation drove a consistent PcG clustering which determined an altered nuclear shape as measured by the decrease in the nuclear volume and area (Fig. 12f-g). Taken together, these findings showed that nuclear architecture is influenced by the PcG-mediated chromatin compartmentalization.

MLL4 constrains mechano-responsiveness of MSCs

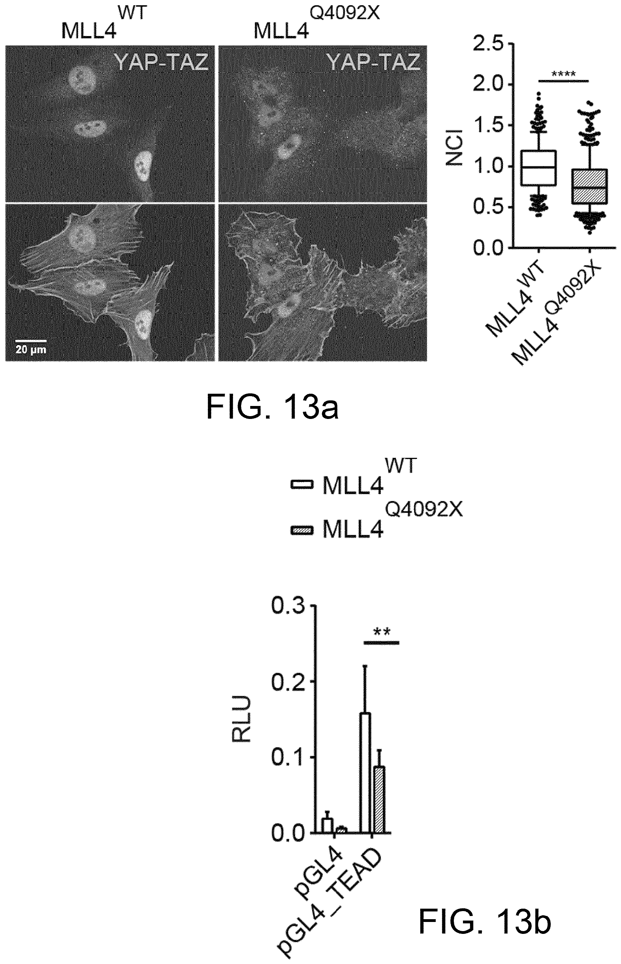

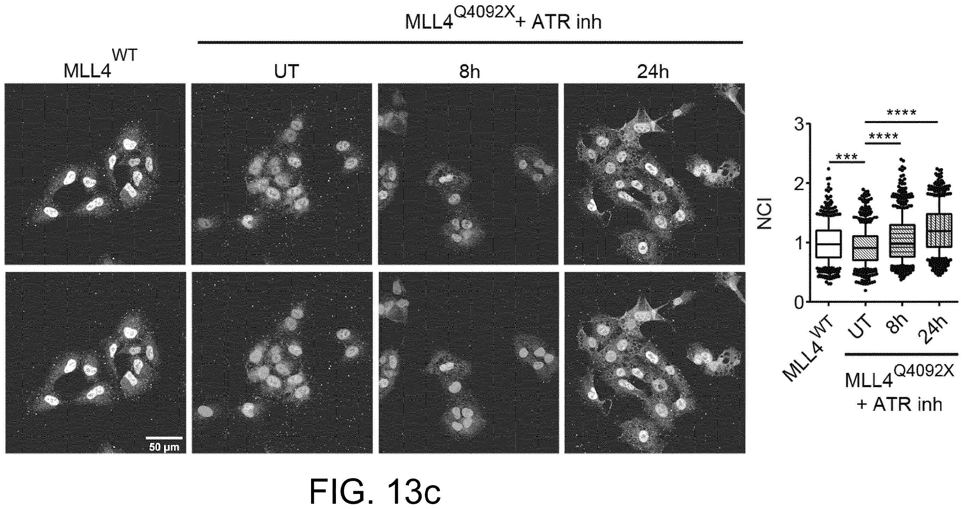

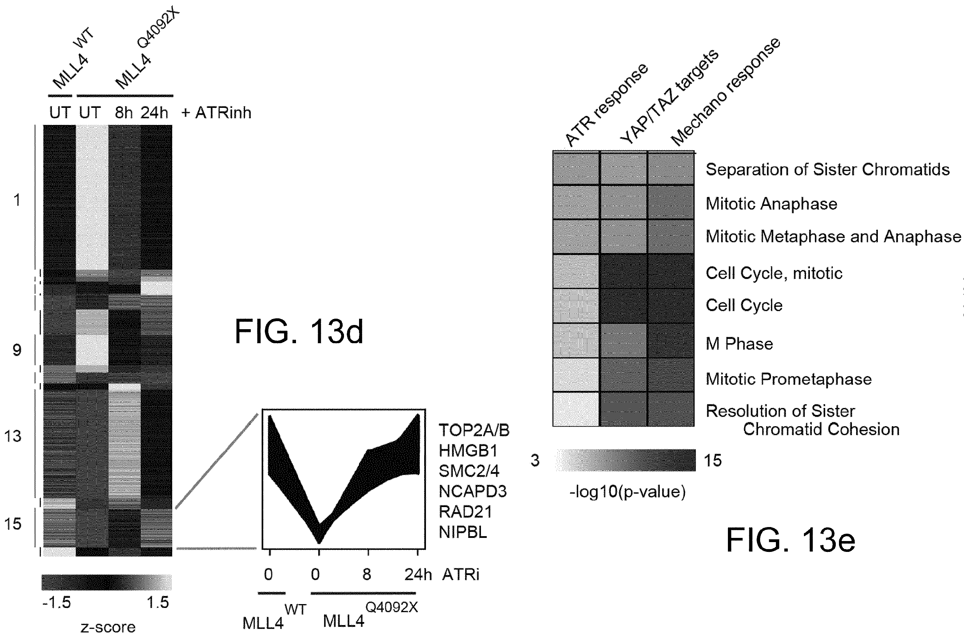

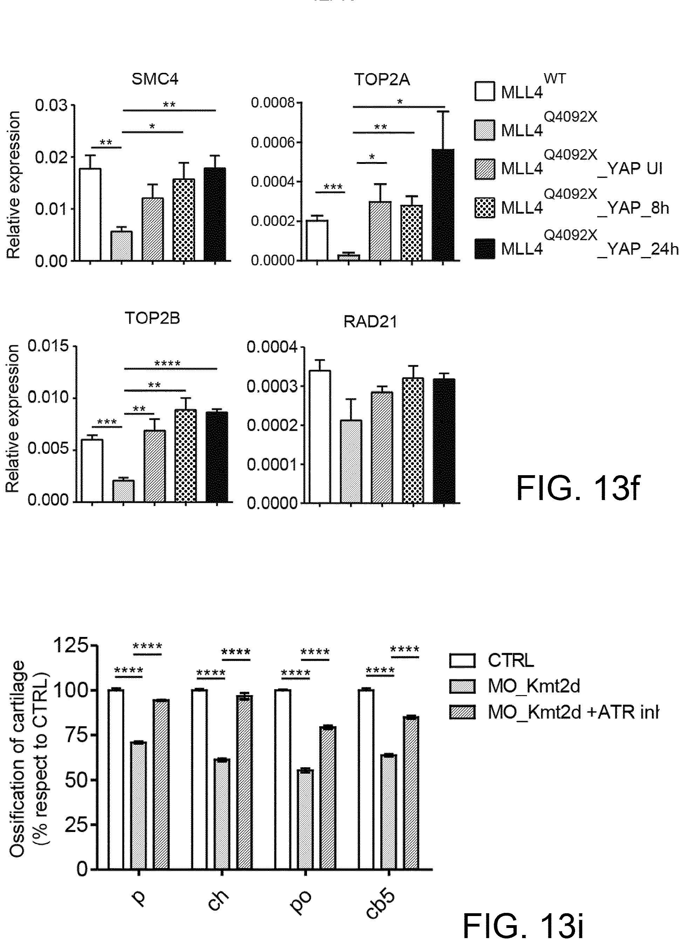

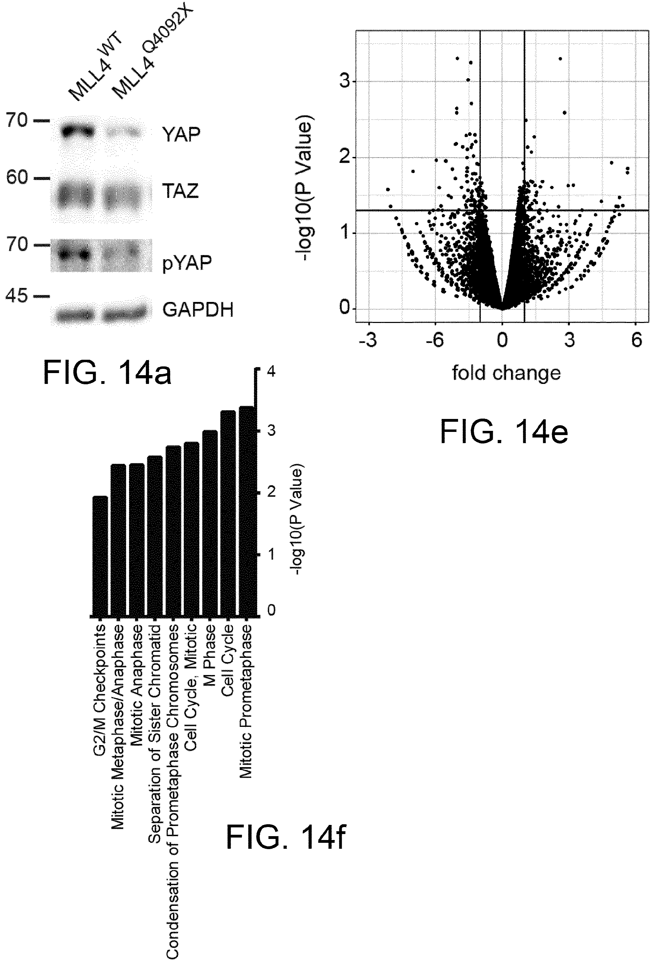

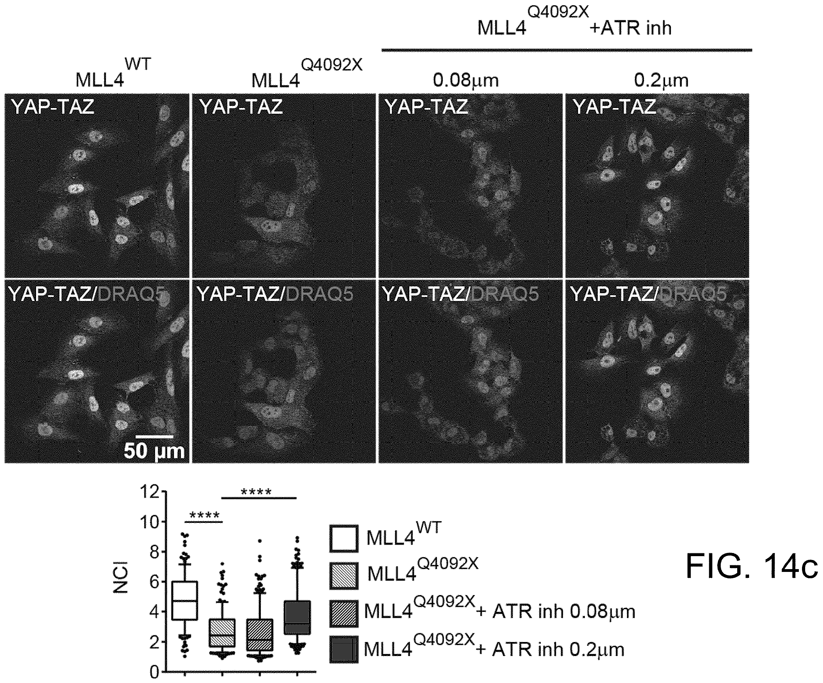

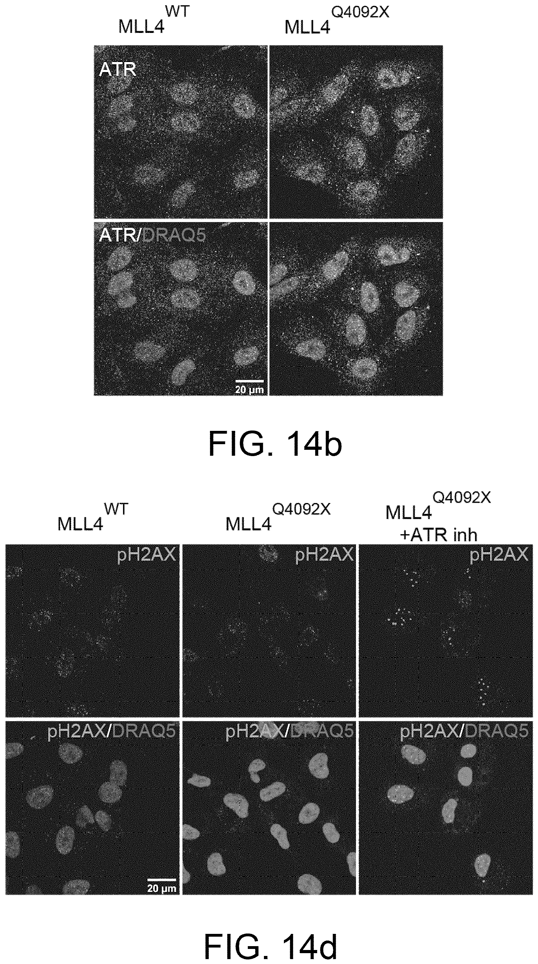

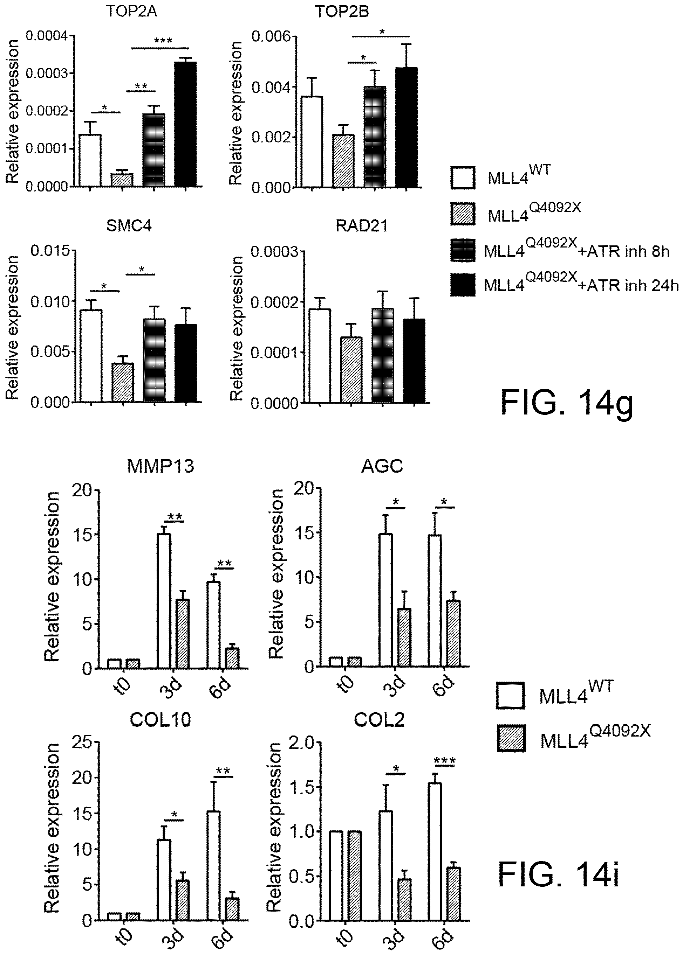

The inventors investigated whether the alterations in nuclear architecture driven by unbalance of transcriptional and PcG condensates would affect mechano-responsiveness of MSCs. To address this point, the inventors first measured the cellular distribution of the mechano-effect YAP1/TAZ whose nuclear accumulation depends on mechanical forces (Elosegui-Artola et al., 2017) (Driscoll et al., 2015) (Dupont et al., 2011). By quantifying the nuclear/cytosolic ratio of YAP/TAZ, the inventors found that maintaining MSCs in sparse condition on stiff substrate induced its nuclear accumulation, in agreement with previous findings (Fig. 13a) (Elosegui-Artola et al., 2017) (Driscoll et al., 2015) (Dupont et al., 2011). When the inventors compared the cellular distribution of YAP/TAZ in WT respect to MLL4$^{Q4092X}$ MSCs, the inventors found that MLL4 LoF was associated with a reduction of nuclear YAP/TAZ (Fig. 13a). Of importance, the mechanical-induced YAP/TAZ nuclear localization was mirrored by an augmented YAP/TAZ transcriptional activity, which was impaired in MLL4$^{Q4092X}$ MSCs (Fig. 13b). Of note, the altered nuclear localization was uncoupled from Hippo pathway activation (Fig. 14a). In order to verify that the reduced YAP/TAZ nuclear shuttling was caused by the altered nuclear mechanics of MLL4$^{Q4092X}$ MSCs, the inventors blocked the nuclear mechanical sensor ATR with its specific inhibitor VE-822 (Fig. 14b and Fig. 14c) (Kumar et al., 2014) (Burgess et al., 2014). The inventors found that treatment of MLL4$^{Q4092X}$ MSCs with ATR inhibitor restored YAP/TAZ nuclear localization (Fig. 13c), without affecting DNA damage response (Fig. 14d). In order to identify the responsive genes to the ATR inhibition in the context of augmented nuclear mechanical stress, the inventors performed gene expression profiling of MSCs at different time points after VE-822 treatment. Differential expression analyses showed that a relatively small subset of genes resulted deregulated in MLL4$^{Q4012X}$ respect to WT MSCs (Fig. 14e-f). Nevertheless, gene clustering analyses identified a subset of ATR-responsive genes whose expression was rescued in MLL4$^{Q4092X}$ MSCs by ATR inhibitor treatment (Fig. 13d). Gene ontology analyses showed that this cluster was enriched for architectural chromatin genes, which are involved in chromatin condensation during mitosis (Fig. 13e, Fig. 14g). Of note, a similar enrichment was retrieved by analyzing both YAP/TAZ target genes as well as mechano-responsive genes (Fig. 13e) (Zanconato et al., 2015) (Provenzano et al., 2009). Together, these findings suggested that the ATR responsive genes could represent downstream targets of the mechanosensor YAP. To test this hypothesis, the inventors manipulated the endogenous level of YAP1 by transiently inducing its overexpression in MLL4$^{Q4092X}$ MSCs. In this setting, the inventors measured an increment of the expression level of architectural chromatin genes such as TOP2A, TOP2B, Condensin factors SMC2/4, and Cohesin component RAD21 (Fig. 13f). These finding indicated that the mechano-sensor ATR modulated the YAP nuclear accumulation and consequently the expression of key factors involved in chromatin organization and compaction.

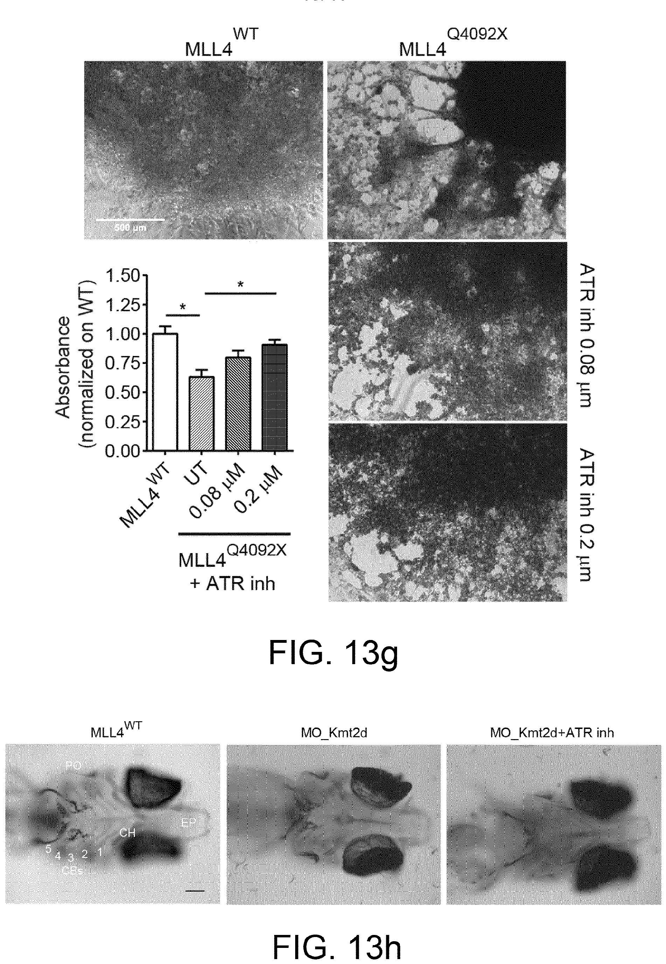

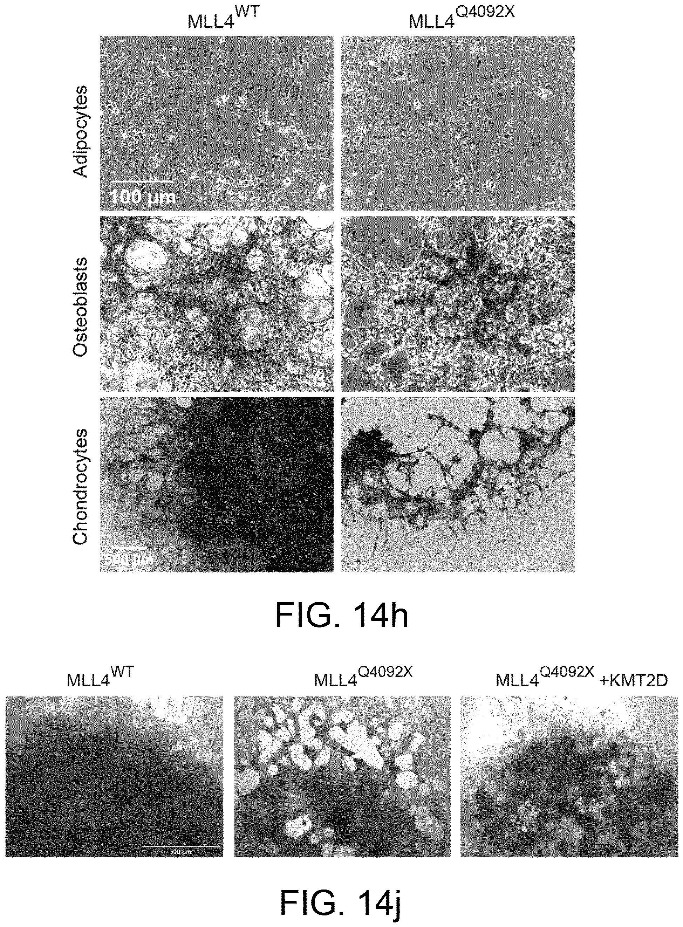

To establish the biological relevance of these results the inventors determined whether MLL4 LoF affected the mechano-responsiveness of MSCs during differentiation. The inventors found that although the differentiation potential of MLL4$^{Q4092X}$ MSCs towards adipocytes was not altered, their commitment towards chondrocytes was strongly affected, while osteogenesis was partially impaired (Fig. 14h). By analyzing the expression of chondrocyte-specific genes, the inventors observed that the LoF of MLL4 altered their pattern at the analyzed time point (Fig. 14i). Of importance, the altered chondrogenesis was rescued by exogenously expressing MLL4 (Fig. 14j). The inventors focused on chondrogenesis to test whether the attenuation of ATR signaling was sufficient to re-establish a proficient differentiation pattern of MLL4$^{Q4092X}$ MSCs. The inventors found that treating MSCs with ATR inhibitor partially rescued the chondrogenic differentiation potential (Fig. 13g) as cells could form 3D cartilage-like network of chondrocytes producing ECM (Fig. 13g).

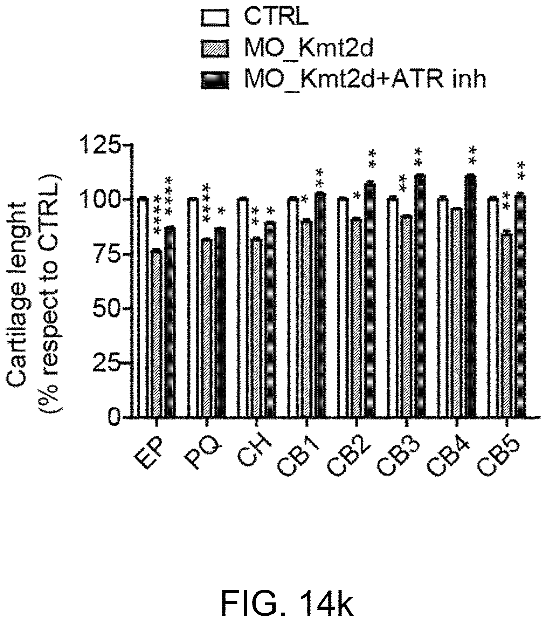

To further support the notion that targeting the nuclear mechano-sensor ATR could re-established the mechanical responsiveness of MSCs in KS, the inventors tested the capacity of ATR inhibitor to rescue chondrogenesis and skeletogenesis *in vivo*. To this end, the inventors developed an *in vivo* model for KS by knocking-down olKmt2d in medaka fish, with a specific morpholino (MO) directed against the ATG initiation codon within the 5' untranslated region (MO-Kmt2d). During early embryogenesis, morphant embryos were indistinguishable from wild-type and control embryos (not shown). However, at later developmental stages a spectrum of morphological cranio-facial anomalies was clearly visible in most of the MO-Kmt2d embryos (73 $\pm$ 5% of 1,600 injected embryos). Growth of cartilage and bone were significantly impaired and culminated in evident shorter length at St40 (Fig. 13h). MO-Kmt2d morphants manifested a smaller head with dysmorphism, resembling clinical features of Kabuki syndrome patients. In particular, cartilage development and mineralization of bones that occurs through endochondral ossification (Ethmoid plate, palatoquadrate, ceratohyal, paired prootics and fifth ceratobranchial) was largely absent or compromised (Fig. 13h and Fig. 14k). Of importance, treatment of st32 Kmt2d morphants with ATR inhibitor was sufficient to restore the chondrogenic and skeleton defects rescuing proper head morphogenesis, without evidence of side effects or toxicity (Fig. 13h and Fig. 14k). Together these results indicated that targeting the mechano-sensor ATR in KS model systems was sufficient to restore proper chondrogenesis and skeletogenesis, both *in vitro* and *in vivo*.

Bibliography

Alberti, S. Phase separation in biology. Curr Biol 27, R1097-R1102 (2017).

Antonacci, G., de Turns, V., Rosa, A., and Ruocco, G. (2018). Background-deflection Brillouin microscopy reveals altered biomechanics of intracellular stress granules by ALS protein FUS. Communications biology 1, 139.

Antonacci, G., Lepert, G., Paterson, C., and Torok, P. (2015). Elastic suppression in Brillouin imaging by destructive interference. Appl Phys Lett 107.

Bolger, A.M., Lohse, M., and Usadel, B. (2014). Trimmomatic: a flexible trimmer for Illumina sequence data. Bioinformatics 30, 2114-2120.

Bugaj, L.J., Choksi, A.T., Mesuda, C.K., Kane, R.S., and Schaffer, D.V. (2013). Optogenetic protein clustering and signaling activation in mammalian cells. Nature methods 10, 249-252.

Burgess, R.C., Burman, B., Kruhlak, M.J., and Misteli, T. (2014). Activation of DNA damage response signaling by condensed chromatin. Cell reports 9, 1703-1717.

Bustin, M., and Misteli, T. (2016). Nongenetic functions of the genome. Science 352, aad6933.

Cho, W.K., Spille, J.H., Hecht, M., Lee, C., Li, C., Grube, V., and Cisse, II (2018). Mediator and RNA polymerase II clusters associate in transcription-dependent condensates. Science 361, 412-415.

Cocciadiferro, D., Augello, B., De Nittis, P., Zhang, J.Y., Mandriani, B., Malerba, N., Squeo, G.M., Romano, A., Piccinni, B., Verri, T., et al. (2018b). Dissecting KMT2D missense mutations in Kabuki syndrome patients. Human molecular genetics 27, 3651-3668.

Conte, I., Hadfield, K.D., Barbato, S., Carrella, S., Pizzo, M., Bhat, R.S., Carissimo, A., Karali, M., Porter, L.F., Urquhart, J., et al. (2015). MiR-204 is responsible for inherited retinal dystrophy associated with ocular coloboma. Proceedings of the National Academy of Sciences of the United States of America 112, E3236-3245.

Dorighi, K.M., Swigut, T., Henriques, T., Bhanu, N.V., Scruggs, B.S., Nady, N., Still, C.D., 2nd, Garcia, B.A., Adelman, K., and Wysocka, J. (2017). Mll3 and Mll4 Facilitate Enhancer RNA Synthesis and Transcription from Promoters Independently of H3K4 Monomethylation. Molecular cell 66, 568-576 e564.

Driscoll, T.P., Cosgrove, B.D., Heo, S.J., Shurden, Z.E., and Mauck, R.L. (2015). Cytoskeletal to Nuclear Strain Transfer Regulates YAP Signaling in Mesenchymal Stem Cells. Biophys J 108, 2783-2793.

Dupont, S., Morsut, L., Aragona, M., Enzo, E., Giulitti, S., Cordenonsi, M., Zanconato, F., Le Digabel, J., Forcato, M., Bicciato, S., et al. (2011). Role of YAP/TAZ in mechanotransduction. Nature 474, 179-183.

Elosegui-Artola, A., Andreu, I., Beedle, A.E.M., Lezamiz, A., Uroz, M., Kosmalska, A.J., Oria, R., Kechagia, J.Z., Rico-Lastres, P., Le Roux, A.L., et al. (2017). Force Triggers YAP Nuclear Entry by Regulating Transport across Nuclear Pores. Cell 171, 1397-1410 e1314.

Gregoretti, F., Cesarini, E., Lanzuolo, C., Oliva, G., and Antonelli, L. (2016). An Automatic Segmentation Method Combining an Active Contour Model and a Classification Technique for Detecting Polycomb-group Proteinsin High-Throughput Microscopy Images. Methods Mol Biol 1480, 181-197.

Kumar, A., Mazzanti, M., Mistrik, M., Kosar, M., Beznoussenko, G.V., Mironov, A.A., Garre, M., Parazzoli, D., Shivashankar, G.V., Scita, G., et al. (2014). ATR mediates a checkpoint at the nuclear envelope in response to mechanical stress. Cell 158, 633-646.

Lehman, N., Mazery, A.C., Visier, A., Baumann, C., Lachesnais, D., Capri, Y., Toutain, A., Odent, S., Mikaty, M., Goizet, C., et al. (2017). Molecular, clinical and neuropsychological study in 31 patients with Kabuki syndrome and KMT2D mutations. Clinical genetics 92, 298-305.

Lepert, G., Gouveia, R.M., Connon, C.J., and Paterson, C. (2016). Assessing corneal biomechanics with Brillouin spectro-microscopy. Faraday discussions 187, 415-428.

Lewis, P.W., Muller, M.M., Koletsky, M.S., Cordero, F., Lin, S., Banaszynski, L.A., Garcia, B.A., Muir, T.W., Becher, O.J., and Allis, C.D. (2013). Inhibition of PRC2 activity by a gain-of-function H3 mutation found in pediatric glioblastoma. Science 340, 857-861.

Micale, L., Augello, B., Maffeo, C., Selicorni, A., Zucchetti, F., Fusco, C., De Nittis, P., Pellico, M.T., Mandriani, B., Fischetto, R., et al. (2014). Molecular analysis, pathogenic mechanisms, and readthrough therapy on a large cohort of Kabuki syndrome patients. Human mutation 35, 841-850.

Nair, S.J., Yang, L., Meluzzi, D., Oh, S., Yang, F., Friedman, M.J., Wang, S., Suter, T., Alshareedah, I., Gamliel, A., et al. (2019). Phase separation of ligand-activated enhancers licenses cooperative chromosomal enhancer assembly. Nature structural & molecular biology 26, 193-203.

Provenzano, P.P., Inman, D.R., Eliceiri, K.W., and Keely, P.J. (2009). Matrix densityinduced mechanoregulation of breast cell phenotype, signaling and gene expression through a FAK-ERK linkage. Oncogene 28, 4326-4343.

Rada-Iglesias, A., Grosveld, F.G., and Papantonis, A. (2018). Forces driving the three-dimensional folding of eukaryotic genomes. Molecular systems biology 14, e8214.

Ricci, M.A., Manzo, C., Garcia-Parajo, M.F., Lakadamyali, M., and Cosma, M.P. (2015). Chromatin fibers are formed by heterogeneous groups of nucleosomes in vivo. Cell 160, 1145-1158.

Rickels, R., Herz, H.M., Sze, C.C., Cao, K., Morgan, M.A., Collings, C.K., Gause, M., Takahashi, Y.H., Wang, L., Rendleman, E.J., et al. (2017). Histone H3K4 monomethylation catalyzed by Trr and mammalian COMPASS-like proteins at enhancers is dispensable for development and viability. Nature genetics 49, 1647-1653.

Sabari, B.R., Dall'Agnese, A., Boija, A., Klein, I.A., Coffey, E.L., Shrinivas, K., Abraham, B.J., Hannett, N.M., Zamudio, A.V., Manteiga, J.C., et al. (2018). Coactivator condensation at super-enhancers links phase separation and gene control. Science 361.

Schuettengruber, B., Bourbon, H.M., Di Croce, L. & Cavalli, G. Genome Regulation by Polycomb and Trithorax: 70 Years and Counting. Cell 171, 34-57 (2017).

Swift, J., Ivanovska, I.L., Buxboim, A., Harada, T., Dingal, P.C., Pinter, J., Pajerowski, J.D., Spinler, K.R., Shin, J.W., Tewari, M., et al. (2013). Nuclear lamin-A scales with tissue stiffness and enhances matrix-directed differentiation. Science 341, 1240104.

Wang, J., Choi, J.M., Holehouse, A.S., Lee, H.O., Zhang, X., Jahnel, M., Maharana, S., Lemaitre, R., Pozniakovsky, A., Drechsel, D., et al. (2018). A Molecular Grammar Governing the Driving Forces for Phase Separation of Prion-like RNA Binding Proteins. Cell 174, 688-699 e616.

Wang, S.P., Tang, Z., Chen, C.W., Shimada, M., Koche, R.P., Wang, L.H., Nakadai, T., Chramiec, A., Krivtsov, A.V., Armstrong, S.A., et al. (2017). A UTX-MLL4-p300 Transcriptional Regulatory Network Coordinately Shapes Active Enhancer Landscapes for Eliciting Transcription. Molecular cell 67, 308-321 e306.

Zambrano, S., De Toma, I., Piffer, A., Bianchi, M.E., and Agresti, A. (2016). NF-kappaB oscillations translate into functionally related patterns of gene expression. eLife 5, e09100.

Zanconato, F., Forcato, M., Battilana, G., Azzolin, L., Quaranta, E., Bodega, B., Rosato, A., Bicciato, S., Cordenonsi, M., and Piccolo, S. (2015). Genome-wide association between YAP/TAZ/TEAD and AP-1 at enhancers drives oncogenic growth. Nature cell biology 17, 1218-1227.

## Claims

1. A compound selected from inhibitors of the Ataxia Telangiectasia and Rad3-related (ATR) protein and inhibitors of the ChkI protein, for use in the therapeutic treatment of a chromatinopathy.

2. A compound for use according to claim 1, which is an inhibitor of the ATR protein selected from the group consisting of VX-970, BAY 1895344, AZD6738, AZ20, EPT-46464 and VE-821.

3. A compound for use according to claim 2, which is VX-970.

4. A compound for use according to claim 1, which is an inhibitor of the Chkl protein selected from the group consisting of GDC-0575, AZD7762, MK-8776, SAR-020106, CCT245737 and PF-477736.

5. A compound for use according to any of claims 1 to 4, wherein the chromatinopathy is selected from the group consisting of Kabuki Syndrome (KS), Kabuki Syndrome 2 (KS 2), Charge Syndrome (CS), Rubinstein-Taybi syndrome (RT) and Cornelia de Lange syndrome (CdL).

6. A compound for use according to claim 5, wherein the chromatinopathy is Kabuki Syndrome (KS).


**Patentansprüche**

1. Verbindung, ausgewählt aus Inhibitoren des Ataxia Telangiectasia-Proteins und des Rad3-bezogenen (ATR) Proteins und Inhibitoren des Chk1-Proteins, zur Verwendung in der therapeutischen Behandlung einer Chromatinopathie.

2. Verbindung zur Verwendung gemäß Anspruch 1, die ein Inhibitor des ATR-Proteins ist, ausgewählt aus der Gruppe bestehend aus VX-970, BAY 1895344, AZD6738, AZ20, EPT-46464 und VE-821.

3. Verbindung zur Verwendung gemäß Anspruch 2, die VX-970 ist.

4. Verbindung zur Verwendung gemäß Anspruch 1, die ein Inhibitor des Chk1-Proteins ist, ausgewählt aus der Gruppe bestehend aus GDC-0575, AZD7762, MK-8776, SAR-020106, CCT245737 und PF-477736.

5. Verbindung zur Verwendung gemäß einem der Ansprüche 1 bis 4, wobei die Chromatinopathie aus der Gruppe bestehend aus dem Kabuki-Syndrom (KS), dem Kabuki-Syndrom 2 (KS 2), dem Charge-Syndrom (CS), dem Rubinstein-Taybi-Syndrom (RT) und dem Cornelia de Lange-Syndrom (CdL) ausgewählt ist.

6. Verbindung zur Verwendung gemäß Anspruch 5, wobei die Chromatinopathie das Kabuki-Syndrom (KS) ist.


**Revendications**

1. Composé sélectionné parmi les inhibiteurs de la protéine ATR (Ataxia Telangiectasia and Rad3-related) et les inhibiteurs de la protéine Chk1, pour son utilisation dans le traitement thérapeutique d'une chromatinopathie.

2. Composé pour son utilisation selon la revendication 1, qui est un inhibiteur de la protéine ATR sélectionné parmi le groupe constitué de VX-970, BAY 1895344, AZD6738, AZ20, EPT-46464 et VE-821.

3. Composé pour son utilisation selon la revendication 2, qui est VX-970.

4. Composé pour son utilisation selon la revendication 1, qui est un inhibiteur de la protéine Chk1 sélectionné parmi le groupe constitué de GDC-0575, AZD7762, MK-8776, SAR-020106, CCT245737 et PF-477736.

5. Composé pour son utilisation selon l'une quelconque des revendications 1 à 4, dans lequel la chromatinopathie est sélectionnée parmi le groupe constitué du syndrome de Kabuki (KS), du syndrome de Kabuki 2 (KS 2), du syndrome de Charge (CS), du syndrome de Rubinstein-Taybi (RT) et du syndrome de Cornelia de Lange (CdL).

6. Composé pour son utilisation selon la revendication 5, dans lequel la chromatinopathie est le syndrome de Kabuki (KS).

FIG. 1a

FIG. 1b

FIG. 1c

FIG. 1d

FIG. 1e

FIG. 1f

FIG. 1g

FIG. 1h

FIG. 2a

H3 Unmod
H3 K4me1
H3 K4me2
H3 K4me3

FIG. 2d

FIG. 2e

## FIG. 2b

## FIG. 2c

FIG. 2f

FIG. 2g

FIG. 3a

FIG. 3b

FIG. 3c

FIG. 3d

FIG. 3e

FIG. 3f

FIG. 3g

FIG. 3h

FIG. 3i

FIG. 3j

FIG. 4a
FIG. 4b
FIG. 4c
FIG. 4d

FIG. 4e

FIG. 4f

mCherry-CRY2

FIG. 4g

FIG. 4h

FIG. 5a

FIG. 5b

FIG. 5c

FIG. 5d

FIG. 5e

FIG. 5h

FIG. 5j

MLL4_PrLD

| 1 µM | 3 µM | 5 µM | 7 µM | 10 µM |
|------|------|------|------|-------|

FIG. 5f

MLL4_PrLD

| 1 µM | 3 µM | 5 µM | 7 µM | 10 µM |
|------|------|------|------|-------|

FIG. 5g

| Pre-stimulation | T0 | 30s | 90s | 240s | 600s |
|-----------------|-----|-----|-----|------|------|

MLL4_PrLD

10 µm

MLL4_PrLDΔQ

FIG. 5i

FIG. 5k

MLL4-PrLD

FIG. 6e

MLL4-PrLD ΔQ

FIG. 6h

FIG. 6a

FIG. 6d

FIG. 6b

COREscore: 61.606

FIG. 6c

FIG. 6f

FIG. 6g

FIG. 7a

FIG. 7b

FIG. 7c

FIG. 7d

FIG. 7e

FIG. 8a

FIG. 8b

FIG. 8c

EP 4 121 166 B1

FIG. 8d

FIG. 8e

FIG. 8f

FIG. 8g

FIG. 8h

FIG. 9a

FIG. 9c

FIG. 9d

FIG. 9b

FIG. 9e

FIG. 9f

FIG. 9g

FIG. 9h

FIG. 9i

FIG. 10a

FIG. 10b

FIG. 10c

FIG. 10d

FIG. 10e

FIG. 11g

FIG. 11a

FIG. 11b

FIG. 11d

FIG. 11c

FIG. 11e

FIG. 11f

FIG. 12a

FIG. 12b

FIG. 12c

FIG. 12d

Pre-stimulation    0    1m    3m    5m    10m    30m

BMI-mCh-CRY2

10 µm

FIG. 12e

BMI-mCh-Cry2    MOCK cells

BMI-mCh-Cry2

Pre-Stimulation

5 µm

BMI-mCh-Cry2

time post-stimulation (hours)

FIG. 12f

Volume

Nuclear area

time post-stimulus (h)

time post-stimulus (h)

--●-- BMI-mCh-Cry2 positive cells
--■-- MOCK cells

FIG. 12g

FIG. 13a

FIG. 13b

FIG. 13c

FIG. 13d

FIG. 13e

FIG. 13f

FIG. 13i

FIG. 13g

FIG. 13h

FIG. 14a

FIG. 14e

FIG. 14f

FIG. 14c

FIG. 14b

FIG. 14d

FIG. 14g

FIG. 14i

FIG. 14h

FIG. 14j

FIG. 14k

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *CHEMICAL ABSTRACTS,* 1232416-25-9
- *CHEMICAL ABSTRACTS,* 1876467-74-1
- *CHEMICAL ABSTRACTS,* 1352226-88-0
- *CHEMICAL ABSTRACTS,* 1233339-22-4
- *CHEMICAL ABSTRACTS,* 1345675-02-6
- *CHEMICAL ABSTRACTS,* 1232410-49-9
- *CHEMICAL ABSTRACTS,* 1196541-47-5
- *CHEMICAL ABSTRACTS,* 860352-01-8
- *CHEMICAL ABSTRACTS,* 891494-63-6
- *CHEMICAL ABSTRACTS,* 1184843-57-9
- *CHEMICAL ABSTRACTS,* 1489389-18-5
- *CHEMICAL ABSTRACTS,* 952021-60-2
- **ALBERTI, S.** Phase separation in biology. *Curr Biol,* 2017, vol. 27, R1097-R1102
- **ANTONACCI, G. ; DE TURNS, V. ; ROSA, A. ; RUOCCO, G.** Background-deflection Brillouin microscopy reveals altered biomechanics of intracellular stress granules by ALS protein FUS. *Communications biology,* 2018, vol. 1, 139
- **ANTONACCI, G. ; LEPERT, G. ; PATERSON, C. ; TOROK, P.** Elastic suppression in Brillouin imaging by destructive interference. *Appl Phys Lett,* 2015, vol. 107
- **BOLGER, A.M. ; LOHSE, M. ; USADEL, B.** Trimmomatic: a flexible trimmer for Illumina sequence data. *Bioinformatics,* 2014, vol. 30, 2114-2120
- **BUGAJ, L.J. ; CHOKSI, A.T. ; MESUDA, C.K. ; KANE, R.S. ; SCHAFFER, D.V.** Optogenetic protein clustering and signaling activation in mammalian cells. *Nature methods,* 2013, vol. 10, 249-252
- **BURGESS, R.C. ; BURMAN, B. ; KRUHLAK, M.J. ; MISTELI, T.** Activation of DNA damage response signaling by condensed chromatin. *Cell reports,* 2014, vol. 9, 1703-1717
- **BUSTIN, M. ; MISTELI, T.** Nongenetic functions of the genome. *Science,* 2016, vol. 352, aad6933
- **CHO, W.K. ; SPILLE, J.H. ; HECHT, M. ; LEE, C. ; LI, C. ; GRUBE, V. ; CISSE, II.** Mediator and RNA polymerase II clusters associate in transcription-dependent condensates. *Science,* 2018, vol. 361, 412-415
- **COCCIADIFERRO, D. ; AUGELLO, B. ; DE NITTIS, P. ; ZHANG, J.Y. ; MANDRIANI, B. ; MALERBA, N. ; SQUEO, G.M. ; ROMANO, A. ; PICCINNI, B. ; VERRI, T. et al.** Dissecting KMT2D missense mutations in Kabuki syndrome patients. *Human molecular genetics,* 2018, vol. 27, 3651-3668
- **CONTE, I. ; HADFIELD, K.D. ; BARBATO, S. ; CARRELLA, S. ; PIZZO, M. ; BHAT, R.S. ; CARISSIMO, A. ; KARALI, M. ; PORTER, L.F. ; URQUHART, J. et al.** MiR-204 is responsible for inherited retinal dystrophy associated with ocular coloboma. *Proceedings of the National Academy of Sciences of the United States of America,* 2015, vol. 112, E3236-3245
- **DORIGHI, K.M. ; SWIGUT, T. ; HENRIQUES, T. ; BHANU, N.V. ; SCRUGGS, B.S. ; NADY, N. ; STILL, C.D., 2ND ; GARCIA, B.A. ; ADELMAN, K. ; WYSOCKA, J.** Mll3 and Mll4 Facilitate Enhancer RNA Synthesis and Transcription from Promoters Independently of H3K4 Monomethylation. *Molecular cell,* 2017, vol. 66, 568-576, e564
- **DRISCOLL, T.P. ; COSGROVE, B.D. ; HEO, S.J. ; SHURDEN, Z.E. ; MAUCK, R.L.** Cytoskeletal to Nuclear Strain Transfer Regulates YAP Signaling in Mesenchymal Stem Cells. *Biophys J,* 2015, vol. 108, 2783-2793
- **DUPONT, S. ; MORSUT, L. ; ARAGONA, M. ; ENZO, E. ; GIULITTI, S. ; CORDENONSI, M. ; ZANCONATO, F. ; LE DIGABEL, J. ; FORCATO, M. ; BICCIATO, S. et al.** Role of YAP/TAZ in mechanotransduction. *Nature,* 2011, vol. 474, 179-183
- **ELOSEGUI-ARTOLA, A. ; ANDREU, I. ; BEEDLE, A.E.M. ; LEZAMIZ, A. ; UROZ, M. ; KOSMALSKA, A.J. ; ORIA, R. ; KECHAGIA, J.Z. ; RICO-LASTRES, P. ; LE ROUX, A.L. et al.** Force Triggers YAP Nuclear Entry by Regulating Transport across Nuclear Pores. *Cell,* 2017, vol. 171, 1397-1410, e1314
- **GREGORETTI, F. ; CESARINI, E. ; LANZUOLO, C. ; OLIVA, G. ; ANTONELLI, L.** An Automatic Segmentation Method Combining an Active Contour Model and a Classification Technique for Detecting Polycomb-group Proteinsin High-Throughput Microscopy Images. *Methods Mol Biol,* 2016, vol. 1480, 181-197
- **KUMAR, A. ; MAZZANTI, M. ; MISTRIK, M. ; KOSAR, M. ; BEZNOUSSENKO, G.V. ; MIRONOV, A.A. ; GARRE, M. ; PARAZZOLI, D. ; SHIVASHANKAR, G.V. ; SCITA, G. et al.** ATR mediates a checkpoint at the nuclear envelope in response to mechanical stress. *Cell,* 2014, vol. 158, 633-646

- LEHMAN, N. ; MAZERY, A.C. ; VISIER, A. ; BAUMANN, C. ; LACHESNAIS, D. ; CAPRI, Y. ; TOUTAIN, A. ; ODENT, S. ; MIKATY, M. ; GOIZET, C. et al. Molecular, clinical and neuropsychological study in 31 patients with Kabuki syndrome and KMT2D mutations. *Clinical genetics,* 2017, vol. 92, 298-305
- LEPERT, G. ; GOUVEIA, R.M. ; CONNON, C.J. ; PATERSON, C. Assessing corneal biomechanics with Brillouin spectro-microscopy. *Faraday discussions,* 2016, vol. 187, 415-428
- LEWIS, P.W. ; MULLER, M.M. ; KOLETSKY, M.S. ; CORDERO, F. ; LIN, S. ; BANASZYNSKI, L.A. ; GARCIA, B.A. ; MUIR, T.W. ; BECHER, O.J. ; ALLIS, C.D. Inhibition of PRC2 activity by a gain-of-function H3 mutation found in pediatric glioblastoma. *Science,* 2013, vol. 340, 857-861
- MICALE, L. ; AUGELLO, B. ; MAFFEO, C. ; SELICORNI, A. ; ZUCCHETTI, F. ; FUSCO, C. ; DE NITTIS, P. ; PELLICO, M.T. ; MANDRIANI, B. ; FISCHETTO, R. et al. Molecular analysis, pathogenic mechanisms, and readthrough therapy on a large cohort of Kabuki syndrome patients. *Human mutation,* 2014, vol. 35, 841-850
- NAIR, S.J. ; YANG, L. ; MELUZZI, D. ; OH, S. ; YANG, F. ; FRIEDMAN, M.J. ; WANG, S. ; SUTER, T. ; ALSHAREEDAH, I. ; GAMLIEL, A. et al. Phase separation of ligand-activated enhancers licenses cooperative chromosomal enhancer assembly. *Nature structural & molecular biology,* 2019, vol. 26, 193-203
- PROVENZANO, P.P. ; INMAN, D.R. ; ELICEIRI, K.W. ; KEELY, P.J. Matrix densityinduced mechanoregulation of breast cell phenotype, signaling and gene expression through a FAK-ERK linkage. *Oncogene,* 2009, vol. 28, 4326-4343
- RADA-IGLESIAS, A. ; GROSVELD, F.G. ; PAPANTONIS, A. Forces driving the three-dimensional folding of eukaryotic genomes. *Molecular systems biology,* 2018, vol. 14, e8214
- RICCI, M.A. ; MANZO, C. ; GARCIA-PARAJO, M.F. ; LAKADAMYALI, M. ; COSMA, M.P. Chromatin fibers are formed by heterogeneous groups of nucleosomes in vivo. *Cell,* 2015, vol. 160, 1145-1158
- RICKELS, R. ; HERZ, H.M. ; SZE, C.C. ; CAO, K. ; MORGAN, M.A. ; COLLINGS, C.K. ; GAUSE, M. ; TAKAHASHI, Y.H. ; WANG, L. ; RENDLEMAN, E.J. et al. Histone H3K4 monomethylation catalyzed by Trr and mammalian COMPASS-like proteins at enhancers is dispensable for development and viability. *Nature genetics,* 2017, vol. 49, 1647-1653
- SABARI, B.R. ; DALL'AGNESE, A. ; BOIJA, A. ; KLEIN, I.A. ; COFFEY, E.L. ; SHRINIVAS, K. ; ABRAHAM, B.J. ; HANNETT, N.M. ; ZAMUDIO, A.V. ; MANTEIGA, J.C. et al. Coactivator condensation at super-enhancers links phase separation and gene control. *Science,* 2018, 361
- SCHUETTENGRUBER, B. ; BOURBON, H.M. ; DI CROCE, L. ; CAVALLI, G. Genome Regulation by Polycomb and Trithorax: 70 Years and Counting. *Cell,* 2017, vol. 171, 34-57
- SWIFT, J. ; IVANOVSKA, I.L. ; BUXBOIM, A. ; HARADA, T. ; DINGAL, P.C. ; PINTER, J. ; PAJEROWSKI, J.D. ; SPINLER, K.R. ; SHIN, J.W. ; TEWARI, M. et al. Nuclear lamin-A scales with tissue stiffness and enhances matrix-directed differentiation. *Science,* 2013, vol. 341, 1240104
- WANG, J. ; CHOI, J.M. ; HOLEHOUSE, A.S. ; LEE, H.O. ; ZHANG, X. ; JAHNEL, M. ; MAHARANA, S. ; LEMAITRE, R. ; POZNIAKOVSKY, A. ; DRECHSEL, D. et al. A Molecular Grammar Governing the Driving Forces for Phase Separation of Prion-like RNA Binding Proteins. *Cell,* 2018, vol. 174, 688-699, e616
- WANG, S.P. ; TANG, Z. ; CHEN, C.W. ; SHIMADA, M. ; KOCHE, R.P. ; WANG, L.H. ; NAKADAI, T. ; CHRAMIEC, A. ; KRIVTSOV, A.V. ; ARMSTRONG, S.A. et al. A UTX-MLL4-p300 Transcriptional Regulatory Network Coordinately Shapes Active Enhancer Landscapes for Eliciting Transcription. *Molecular cell,* 2017, vol. 67, 308-321, e306
- ZAMBRANO, S. ; DE TOMA, I. ; PIFFER, A. ; BIANCHI, M.E. ; AGRESTI, A. NF-kappaB oscillations translate into functionally related patterns of gene expression. *eLife,* 2016, vol. 5, e09100
- ZANCONATO, F. ; FORCATO, M. ; BATTILANA, G. ; AZZOLIN, L. ; QUARANTA, E. ; BODEGA, B. ; ROSATO, A. ; BICCIATO, S. ; CORDENONSI, M. ; PICCOLO, S. Genome-wide association between YAP/TAZ/TEAD and AP-1 at enhancers drives oncogenic growth. *Nature cell biology,* 2015, vol. 17, 1218-1227